# EUROPÄISCHE PATENTANMELDUNG

(11) **EP 1 992 614 A1**
(43) Veröffentlichungstag der Anmeldung: **19.11.2008**
(21) Anmeldenummer: 07009766.2
(22) Anmeldetag: 16.05.2007
(51) Int. Cl.: C07D 207/36, C07D 209/96, C07D 405/04, C07D 487/04, A01N 43/36, A01N 43/38, A01N 43/90

(54) **3-(2-Alkoxy-phenyl)-substituierte Tetramate**

(71) Anmelder: Bayer CropScience Aktiengesellschaft, 40789 Monheim (DE)
(72) Erfinder: Die Erfindernennung liegt noch nicht vor

(57) **Zusammenfassung**

Die Erfindung betrifft neue 4-(2-Alkoxy-phenyl)-substituierte Tetramate der Formel (I) 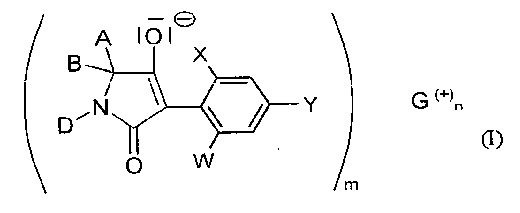 in welcher
W, X, Y, G, m, n, A, B, und D die oben angegebene Bedeutung haben, Verfahren zu ihrer Herstellung und ihre Verwendung als Schädlingsbekämpfungsmittel und/oder Herbizide.

Auch Gegenstand der Erfindung sind selektiv herbizide Mittel, die 3-(2-Alkoxy-phenyl)substituierte Tetramate einerseits und eine die Kulturpflanzenverträglichkeit verbessende Verbindung andererseits enthalten.

## Beschreibung

Die vorliegende Erfindung betrifft neue 3-(2-Alkoxy-phenyl)-substituierte Tetramate, mehrere Verfahren zu ihrer Herstellung und ihre Verwendung als Schädlingsbekämpfungsmittel und/oder Herbizide. Auch Gegenstand der Erfindung sind selektiv herbizide Mittel, die 3-(2-Alkoxy-phenyl)-substituierte Tetramate einerseits und eine die Kulturpflanzenverträglichkeit verbessernde Verbindung andererseits enthalten.

Die vorliegende Erfindung betrifft weiterhin die Steigerung der Wirkung von Pflanzenschutzmitteln enthaltend insbesondere 3-2-(Alkoxy-phenyl)-substituierte Tetramate, durch die Zugabe von Ammonium- oder Phosphoniumsalzen und gegebenefalls Penetrationsförderern, die entsprechenden Mittel, Verfahren zur ihrer Herstellung und ihre Anwendung im Pflanzenschutz als Insektizide und/oder Akarizide und/oder zur Verhinderung von unerwünschten Pflanzenwuchs.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599, EP-A-415 211 und JP-A-12-053 670) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 94/01 997, WO 95/26 954, WO 95/20 572, EP-A-0 668 267, WO 96/25 395, WO 96/35 664, WO 97/01 535, WO 97/02 243, WO 97/36 868, WO 97/43275, WO 98/05638, WO 98/06721, WO 98/25928, WO 99/24437, WO 99/43649, WO 99/48869 und WO 99/55673, WO 01/17972, WO 01/23354, WO 01/74770, WO 03/013249, WO 03/062244, WO 2004/007448, WO 2004/024 688, WO 04/065366, WO 04/080962, WO 04/111042, WO 05/044791, WO 05/044796, WO 05/048710, WO 05/049596, WO 05/066125, WO 05/092897, WO 06/000355, WO 06/029799, WO 06/056281, WO 06/056282, WO 06/089633 und DE-A-05051325. Außerdem sind ketalsubstituierte 1-H-Arylpyrrolidin-2,4-dione aus WO 99/16748 und (spiro)-ketalsubstituierte N-Alkoxy-alkoxy-substituierte Aryl-pyrrolidindione aus JP-A-14 205 984 und Ito M. et. al Bioscience, Biotechnology and Biochemistry 67, 1230-1238, (2003) bekannt. Der Zusatz von Safenem zu Ketoenolen ist ebenfalls prinzipiell aus der WO 03/013249 bekannt. Außerdem sind aus WO 06/024411 herbizide Mittel enthaltend Ketoenole bekannt.

Die herbizide und/oder akarizide und/oder insektizide Wirksamkeit und/oder Wirkungsbreite und/oder die Pflanzenverträglichkeit der bekannten Verbindungen, insbesondere gegenüber Kulturpflanzen, ist jedoch nicht immer ausreichend.

Es wurden nun neue Verbindungen der Formel (I) gefunden 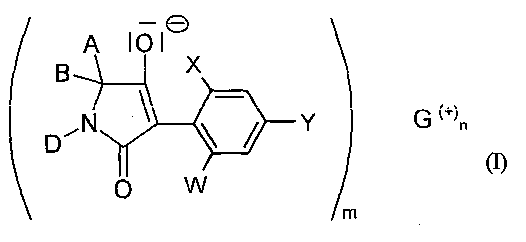 in welcher
- X: für Alkoxy, Halogenalkoxy, Alkoxyakyloxy, Alkoxy-bis-alkyloxy oder gegebenenfalls substituiertes Cycloalkyl-alkandyl-oxy, welches gegebenenfalls durch Heteroatome unterbrochen sein kann, steht,
- W: f¨ür Alkyl steht,
- Y: für Chlor, Brom oder Iod steht,
- G: für ein Metallionenäquivalent steht,
- m: für die Zahl 1 oder 2 steht,
- n: für die Zahl 1 oder 2 steht,
- A: für Wasserstoff, jeweils gegebenenfalls durch Halogen substituiertes Alkyl, Alkenyl, Alkoxyalkyl, Alkylthioalkyl oder Cycloalkyl steht,
- B: für Wasserstoff, Alkyl oder Alkoxyalkyl steht, oder
- A und B: gemeinsam mit dem Kohlenstoffatom an das sie gebunden sind, für einen gesättigten oder ungesättigten, gegebenenfalls mindestens ein Heteroatom enthaltenden unsubstituierten oder substituierten Cyclus stehen,
- D: für Wasserstoff oder einen gegebenenfalls substituierten Rest aus der Reihe Alkyl, Alkenyl, Alkinyl, Alkoxyalkyl, gesättigtes oder ungesättigtes Cycloalkyl, in welchem gegebenenfalls eines oder mehrere Ringglieder durch Heteroatome ersetzt sind, Arylalkyl, Aryl, Hetarylalkyl oder Hetaryl steht oder
- A und D: gemeinsam mit den Atomen an die sie gebunden sind für einen gesättigten oder ungesättigten und gegebenenfalls mindestens ein Heteroatom enthaltenden, im A,D-Teil unsubstituierten oder substituierten Cyclus stehen.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Tautomeren- und Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren und tautomeren Verbindungen gemeint sind.

Weiterhin wurde gefunden, dass man die neuen Verbindungen der Formel (I) nach einem der im folgenden beschriebenen Verfahren erhält:
(A) Man erhält substituierte 3-(2-Alkoxy-phenyl)-substituierte Tetramate der Formel (I) 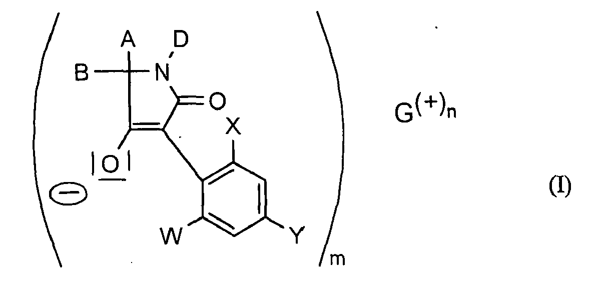 in welcher
   A, B, D, G, m, n, W, X und Y die oben angegebenen Bedeutungen haben,
   wenn man
   N-Acylaminosäureester der Formel (II) 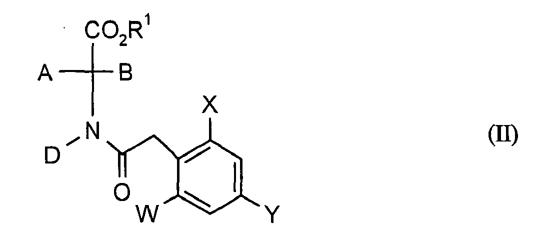 in welcher
   A, B, D, W, X und Y die oben angegebenen Bedeutungen haben,
   und
   R¹ für Alkyl (bevorzugt C₁-C₆-Alkyl) steht,
   in Gegenwart eines Verdünnungsmittels und in Gegenwart einer Metallbase intramolekular kondensiert.
(B) Weiterhin wurde gefunden, dass man Verbindungen der oben gezeigten Formel (I), in welcher A, B, D, G, m, n, W, X und Y die oben angegebenen Bedeutungen haben, erhält, wenn man Verbindungen der Formel (I'), 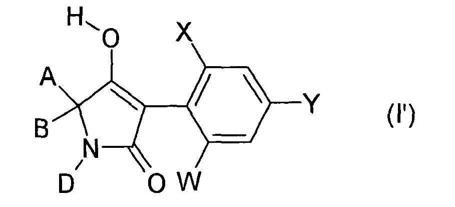
in welchen A, B, D, W, X und Y die oben angegebenen Bedeutungen haben, jeweils
mit Metallverbindungen der Formeln (III) oder (IV)

G(OR²)ₙ (III) ,

G(H)ₙ (IV)

in welchen
- G: für ein ein- oder zweiwertiges Metall (bevorzugt ein Alkali- oder Erdalkalimetall wie Lithium, Natrium, Kalium, Cäsium, Magnesium oder Calcium),
- n: für die Zahl 1 oder 2 und
- R²: für Wasserstoff oder Alkyl (bevorzugt C₁-C₈-Alkyl) steht,
gegebenenfalls in Gegenwart eines Verdünnungsmittels umsetzt.

Weiterhin wurde gefunden, dass die neuen Verbindungen der Formel (I) eine sehr gute Wirksamkeit als Schädlingsbekämpfungsmittel, vorzugsweise als Insektizide, Akarizide und/oder Herbizide aufweisen.

Überraschenderweise wurde nun auch gefunden, dass bestimmte substituierte, cyclische Ketoenole bei gemeinsamer Anwendung mit den im weiteren beschriebenen, die Kulturpflanzen-Verträglichkeit verbessernden Verbindungen (Safenern/Antidots) ausgesprochen gut die Schädigung der Kulturpflanzen verhindern und besonders vorteilhaft als breit wirksame Kombinationspräparate zur selektiven Bekämpfung von unerwünschten Pflanzen in Nutzpflanzenkulturen, wie z.B. in Getreide aber auch Mais, Soja und Reis, verwendet werden können.

Gegenstand der Erfindung sind auch selektiv-herbizide Mittel enthaltend einen wirksamen Gehalt an einer Wirkstoffkombination umfassend als Komponenten
(a') mindestens ein 3-(2-Alkoxy-phenyl)-substituiertes Tetramat der Formel (I), in welcher A, B, D, G, m, n, W, X und Y die oben angegebene Bedeutung haben
   und
(b') zumindest eine die Kulturpflanzen-Verträglichkeit verbessernde Verbindung aus der folgenden Gruppe von Verbindungen:

4-Dichloracetyl-1-oxa-4-aza-spiro[4.5]-decan (AD-67, MON-4660), 1-Dichloracetyl-hexahydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methylhexylester) (Cloquintocet-mexyl - vgl. auch verwandte Verbindungen in EP-A-86750, EP-A-94349, EP-A-191736, EP-A-492366), 3-(2-Chlor-benzyl)-1-(1-methyl-1-phenyl-ethyl)-harnstoff (Cumyluron), α-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methylphenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlor-phenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluorinethyl-thiazol-5-carbonsäure-phenylmethylester (Flurazole), 4-Chlor-N-(1,3-dioxolan-2-yl-methoxy)-a-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyloxy)-essigsäure (MCPA), 2-(4-Chlor-o-tolyloxy)-propionsäure (Mecoprop), Diethyl-1-(2,4-dichlorphenyl)-4,5-dihydro-5-methyl-1H-pyrazol-3,5-dicarboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyl-oxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlorbenzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-ylester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-diallylester, 5-Chlor-chüiolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlor-phenoxy-essigsäure, 3,3'-Dimethyl-4-methoxy-benzophenon, 1-Brom-4-chlormethyl-sulfonyl-benzol, 1-[4-(N-2-Methoxybenzoylsulfamoyl)-phenyl]-3-methyl-harnstoff (alias N-(2-Methoxy-benzoyl)-4-[(methylamino-carbonyl)-amino]-benzolsulfonamid), 1-[4-(N-2-Methoxy-benzoylsulfamoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfonamid,
und/oder eine der folgenden durch allgemeine Formeln definierten Verbindungen
der allgemeinen Formel (IIa) 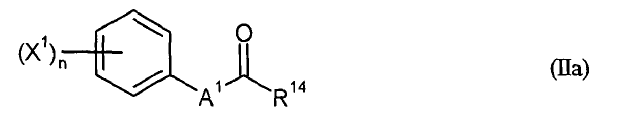 oder der allgemeinen Formel (IIb) 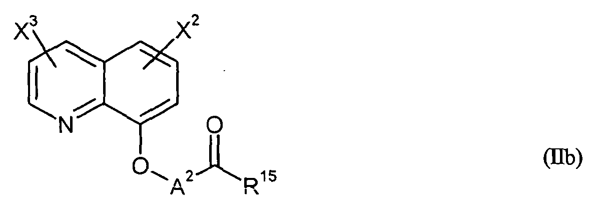 oder der Formel (IIc) 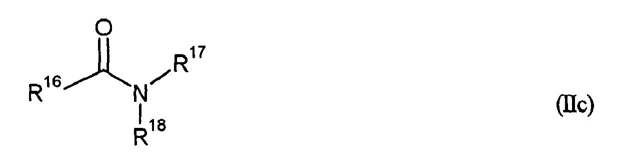 wobei
- n: für eine Zahl zwischen 0 und 5 steht,
- A¹: für eine der nachstehend skizzierten divalenten heterocyclischen Gruppierungen steht, 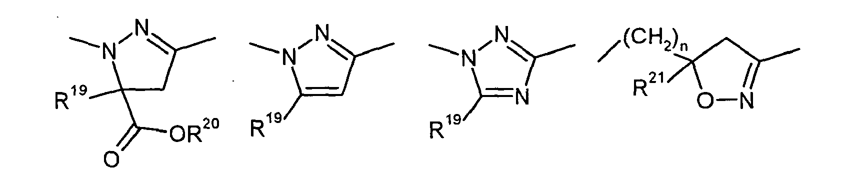
- n: für eine Zahl zwischen 0 und 5 steht,
- A²: für gegebenenfalls durch C₁-C₄-Alkyl und/oder C₁-C₄-Alkoxy-carbonyl substituiertes Alkandiyl mit 1 oder 2 Kohlenstoffatomen steht,
- R¹⁴: für Hydroxy, Mercapto, Amino, C₁-C₆-Alkoxy, C₁-C₆-Alkylthio, C₁-C₆-Alkylamino oder Di-(C₁-C₄-alkyl)-amino steht,
- R¹⁵: für Hydroxy, Mercapto, Amino, C₁-C₆-Akoxy, C₁-C₆-Alkylthio, C₁-C₆-Akylwnino oder Di-(C₁-C₄-alkyl)-amino steht,
- R¹⁶: für jeweils gegebenenfalls durch Fluor, Chlor und/oder Brom substituiertes C₁-C₄-Akyl steht,
- R¹⁷: für Wasserstoff, jeweils gegebenenfalls durch Fluor, Chlor und/oder Brom substituiertes C₁-C₆-Alkyl, C₂-C₆-Akenyl oder C₂-C₆-Allanyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
- R¹⁸: für Wasserstoff, jeweils gegebenenfalls durch Fluor, Chlor und/oder Brom substituiertes C₁-C₆-Alkyl, C₂-C₆-Alkenyl oder C₂-C₆-Akinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl, oder zusammen mit R¹⁷ für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carboxyclus bilden, substituiertes C₃-C₆-Alkmdiyl oder C₂-C₅-Oxaalkandiyl steht,
- R¹⁹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
- R²⁰: für Wasserstoff, gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
- R²¹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
- X¹: für Nitro, Cyano, Halogen, C₁-C₄-Alkyl, C₁-C₄-Halogenalkyl, C₁-C₄-Alkoxy oder C₁-C₄-Halogenalkoxy steht,
- X²: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
- X³: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
und/oder die folgenden durch allgemeine Formeln definierten Verbindungen
der allgemeinen Formel (IId) 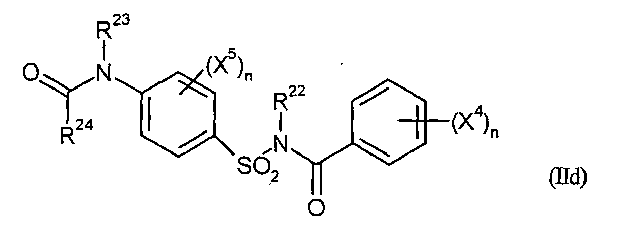 oder der allgemeinen Formel (IIe) 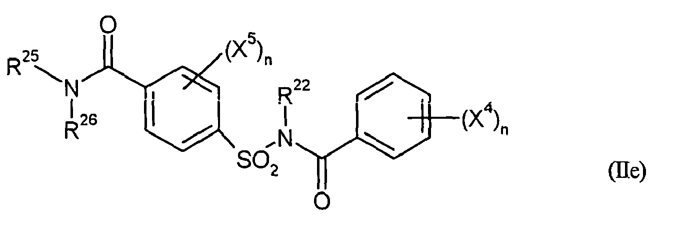 wobei
- n: für eine Zahl zwischen 0 und 5 steht,
- R²²: für Wasserstoff oder C₁-C₄-Akyl steht,
- R²³: für Wasserstoff oder C₁-C₄-Alkyl steht,
- R²⁴: für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁-C₄-Alkyl substituiertes C₃-C₆-Cycloalkyl, C₃-C₆-Cycloalkyloxy, C₃-C₆-Cycloalkylthio oder C₃-C₆-Cycloalkylamino steht,
- R²⁵: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
- R²⁶: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄-Halogenalkoxy substituiertes Phenyl steht, oder zusammen mit R²⁵ für jeweils gegebenenfalls durch C₁-C₄-Akyl substituiertes C₂-C₆-Alkandiyl oder C₂-C₅-Oxaalkandiyl steht,
- X⁴: für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht, und
- X⁵: für Nitro, Cyano, Carboxy, Carbamoyl, Formyl, Sulfamoyl, Hydroxy, Amino, Halogen, C₁-C₄-Alkyl, C₁-C₄-Halogenakyl, C₁-C₄-Alkoxy oder C₁-C₄-Halogenalkoxy steht.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- X: steht bevorzugt für C₁-C₆-Alkoxy, C₁-C₆-Halogenalkoxy, C₁-C₄-Alkoxy-C₂-C₄-alkyloxy, C₁-C₄-Alkoxy-bis-C₂-C₄-alkyloxy oder gegebenenfalls einfach bis dreifach durch Fluor, Chlor, C₁-C₃-Alkyl oder C₁-C₃-Alkoxy substituiertes C₃-C₆-Cycloalkyl-C₁-C₃-alkandiyl-oxy, worin gegebenenfalls eine Methylengruppe des Rings durch Sauerstoff oder Schwefel unterbrochen sein kann,
- W: steht bevorzugt für C₁-C₆-Alkyl, W
- Y: steht bevorzugt für Chlor, Brom oder Iod,
- G: steht bevorzugt für Lithium, Natrium, Kalium, Cäsium, Magnesium-Halogen-Kationen, Magnesium oder Calcium,
- m: steht bevorzugt für die Zahlen 1 oder 2,
- n: steht bevorzugt für die Zahlen 1 oder 2,
- A: steht bevorzugt für Wasserstoff oder jeweils gegebenenfalls durch Halogen substituiertes C₁-C₁₂-Alkyl, C₃-C₈-Alkenyl, C₁-C₁₀-Alkoxy-C₁-C₈-alkyl, C₁-C₁₀-Alkylthio-Cₗ-C₆-alkyl oder Cyclopropyl,
- B: steht bevorzugt für Wasserstoff, C₁-C₁₂-Alkyl oder C₁-C₈-Alkoxy-C₁-C₆-alkyl oder
- A, B: und das Kohlenstoffatom an das sie gebunden sind, stehen bevorzugt für gesättigtes C₃-C₁₀-Cycloalkyl oder ungesättigtes C₅-C₁₀-Cycloalkyl, worin gegebenenfalls ein Ringglied durch Sauerstoff oder Schwefel ersetzt ist und welche gegebenenfalls einfach oder zweifach durch C₁-C₈-Alkyl, C₃-C₁₀-Cycloalkyl, C₁-C₈-Halogenalkyl, C₁-C₈-Alkoxy, C₃-C₆-Cycloalkyl-C₁-C₃-alkoxy, C₁-C₈-Alkylthio, Halogen oder Phenyl substituiert sind oder
- A, B: und das Kohlenstoffatom, an das sie gebunden sind, stehen bevorzugt für C₃-C₆-Cycloalkyl, welches durch eine gegebenenfalls ein oder zwei nicht direkt benachbarte Sauerstoffund/oder Schwefelatome enthaltende gegebenenfalls durch C₁-C₄-Alkyl substituierte Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet oder
- A, B: und das Kohlenstoffatom, an das sie gebunden sind, stehen bevorzugt für C₃-C₈-Cycloalkyl oder C₅-C₈-Cycloalkenyl, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₂-C₆-Alkandiyl, C₂-C₆-Alkendiyl oder C₄-C₆-Alkandiendiyl stehen, worin gegebenenfalls eine Methylengruppe durch Sauerstoff oder Schwefel ersetzt ist,
- D: steht bevorzugt für Wasserstoff, jeweils gegebenenfalls durch Halogen substituiertes C₁-C₁₂-Alkyl, C₃-C₈-Alkenyl, C₃-C₈-Alkinyl, C₁-C₁₀-Alkoxy-C₂-C₈-alkyl, gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls ein Ringglied durch Sauerstoff oder Schwefel ersetzt ist oder
- A und D: stehen gemeinsam bevorzugt für jeweils gegebenenfalls substituiertes C₃-C₆-Alkandiyl oder C₃-C₆-Alkendiyl, worin gegebenenfalls eine Methylengruppe durch eine Carbonylgruppe, Sauerstoff oder Schwefel ersetzt ist und
wobei als Substituenten jeweils in Frage kommen:
Halogen, Hydroxy, Mercapto oder jeweils gegebenenfalls durch Halogen substituiertes C₁-C₁₀- Alkyl, C₁-C₆-Alkoxy, C₁-C₆-Alkylthio, C₃-C₇-Cycloalkyl, Phenyl oder Benzyloxy, oder eine weitere C₃-C₆-Alkandiylgruppierung, C₃-C₆-Alkendiylgruppierung oder eine Butadienylgruppierung, die gegebenenfalls durch C₁-C₆-Alkyl substituiert ist oder in der gegebenenfalls zwei benachbarte Substituenten mit den Kohlenstoffatomen, an die sie gebunden sind, einen weiteren gesättigten oder ungesättigten Cyclus mit 5 oder 6 Ringatomen bilden stehen A und D dann gemeinsam mit den Atomen, an die sie gebunden sind beispielsweise für die weiter unten genannten Gruppen AD-1 bis AD-10, der gegebenenfalls Sauerstoff oder Schwefel enthalten kann, oder worin gegebenenfalls eine der folgenden Gruppen 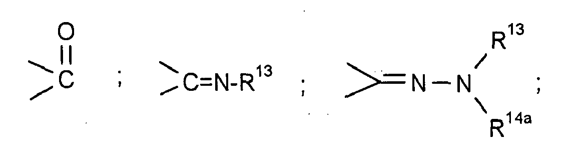 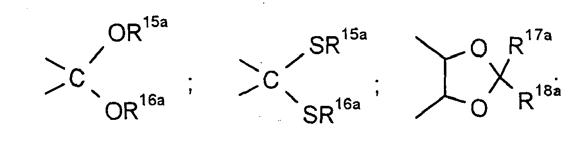 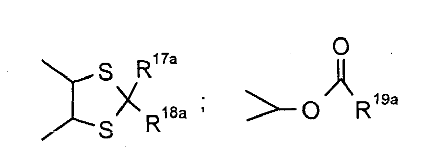 oder 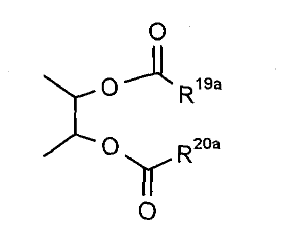 enthalten ist.

In den als bevorzugt genannten Restedefinitionen steht Halogen für Fluor, Chlor, Brom und Jod, insbesondere für Fluor, Chlor und Brom.
- X: steht besonders bevorzugt für C₁-C₄-Alkoxy, C₁-C₄-Halogenalkoxy, C₁-C₃-Alkoxy-C₂-C₃-alkyloxy, C₁-C₂-Alkoxy-bis-C₂-C₃-alkyloxy oder C₃-C₆-Cycloalkyl-C₁-C₂-alkandiyloxy, worin gegebenenfalls eine Methylengruppe des Rings durch Sauerstoff unterbrochen sein kann,
- W: steht besonders bevorzugt für C₁-C₃-Alkyl,
- Y: steht besonders bevorzugt für Chlor oder Brom,
- G: steht besonders bevorzugt für Lithium, Natrium, Kalium, Cäsium, Magnesium-chloridkation, Magnesium-bromid-kation, Magnesium-jodid-kation, Magnesium oder Calcium,
- m: steht besonders bevorzugt für die Zahlen 1 oder 2,
- n: steht besonders bevorzugt für die Zahlen 1 oder 2,
- A: steht besonders bevorzugt für Wasserstoff, jeweils gegebenenfalls einfach bis dreifach durch Fluor oder Chlor substituiertes C₁-C₆-Alkyl, C₁-C₄-Alkoxy-C₁-C₂-alkyl oder Cyclopropyl,
- B: steht besonders bevorzugt für Wasserstoff, C₁-C₄-Alkyl oder C₁-C₂-Alkoxyl-C₁-C₂-alkyl oder
- A, B: und das Kohlenstoffatom an das sie gebunden sind, stehen besonders bevorzugt für gesättigtes oder ungesättigtes C₅-C₇-Cyloalkyl, worin gegebenenfalls ein Ringglied durch Sauerstoff oder Schwefel ersetzt ist und welches gegebenenfalls einfach bis zweifach durch C₁-C₆-Alkyl, Trifluormethyl, C₁-C₆-Alkoxy oder C₃-C₆-Cycloalkyl-C₁-C₂-alkoxy,
- A, B: und das Kohlenstoffatom, an das sie gebunden sind, stehen besonders bevorzugt für C₅-C₆-Cycloalkyl, welches durch eine gegebenenfalls ein oder zwei nicht direkt benachbarte Sauerstoff- oder Schwefelatome enthaltende gegebenenfalls durch Methyl oder Ethyl substituierte Alkylendiyl- oder durch eine Alkylendioxyl- oder durch eine Alkylendithiol-Gruppe substituiert ist, die mit dem Kohlenstoffatom, an das sie gebunden ist, einen weiteren fünf-oder sechsgliedrigen Ring bildet,
- A, B: und das Kohlenstoffatom, an das sie gebunden sind, stehen besonders bevorzugt für C₃-C₆-Cycloalkyl oder C₅-C₆-Cycloalkenyl, in welchen zwei Substituenten gemeinsam mit den Kohlenstoffatomen, an die sie gebunden sind, für jeweils gegebenenfalls durch C₁-C₂-Alkyl oder C₁-C₂-Alkoxy substituiertes C₂-C₄-Alkandiyl, C₂-C₄-Alkendiyl oder Butadiendiyl stehen,
- D: steht besonders bevorzugt für Wasserstoff, für jeweils gegebenenfalls einfach bis dreifach durch Fluor substituiertes C₁-C₆-Alkyl, C₃-C₆-Alkenyl, C₁-C₄-Alkoxy-C₂-C₃-alkyl, für gegebenenfalls einfach bis zweifach durch C₁-C₄-Alkyl, C₁-C₄-Alkoxy oder C₁-C₂-Halogenalkyl substituiertes C₃-C₆-Cycloalkyl,
- A und D: stehen gemeinsam besonders bevorzugt für gegebenenfalls einfach bis zweifach substituiertes C₃-C₅-Alkandiyl, in welchem eine Methylengruppe gegebenenfalls durch Sauerstoff oder Schwefel ersetzt sein kann, wobei als Substituenten C₁-C₂-Alkyl oder C₁-C₂-Alkoxy in Frage kommen oder
- A und D: stehen gemeinsam mit den Atomen, an die sie gebunden sind, für eine der Gruppen AD-1 bis AD-10:

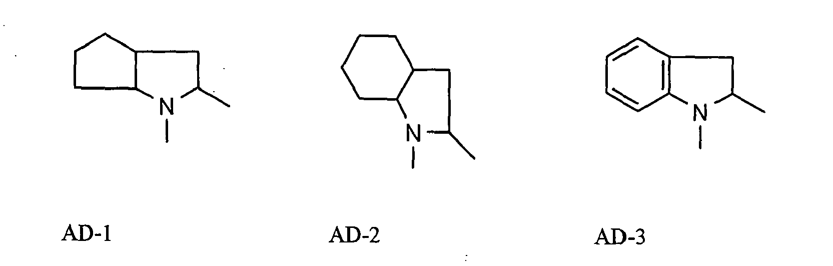

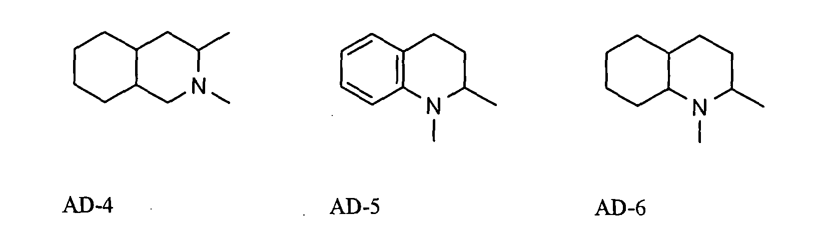

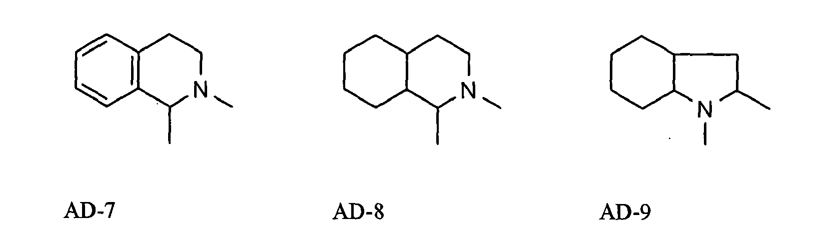

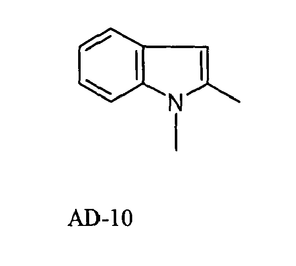

In den als besonders bevorzugt genannten Restedefinitionen steht Halogen für Fluor, Chlor und Brom, insbesondere für Fluor und Chlor.
- X: steht ganz besonders bevorzugt für Methoxy, Ethoxy, n-Propoxy, iso-Propoxy, n-Butoxy, iso-Butoxy, sec-Butoxy, Methoxy-ethyloxy, Ethoxy-ethyloxy, Cyclopropyl-methoxy, Cyclopentyl-methoxy oder Cyclohexyl-methoxy,
- W: steht ganz besonders bevorzugt für Methyl oder Ethyl,
- Y: steht ganz besonders bevorzugt für Chlor oder Brom,
- G: steht ganz besonders bevorzugt für Lithium, Natrium, Kalium, Cäsium, Magnesium-bromid-kation, Magnesium oder Calcium,
- m: steht ganz besonders bevorzugt für die Zahlen 1 oder 2,
- n: steht ganz besonders bevorzugt für die Zahlen 1 oder 2,
- A: steht ganz besonders bevorzugt für Wasserstoff, jeweils gegebenenfalls einfach bis dreifach durch Fluor substituiertes C₁-C₄-Alkyl oder C₁-C₂-Alkoxy-C₁-C₂-alkyl oder Cyclopropyl,
- B: steht ganz besonders bevorzugt für Wasserstoff, Methyl oder Ethyl oder
- A, B: und das Kohlenstoffatom an das sie gebunden sind, stehen ganz besonders bevorzugt für gesättigtes C₅-C₆-Cycloalkyl, in welchem gegebenenfalls ein Ringglied durch Sauerstoff oder Schwefel ersetzt ist und weiches gegebenenfalls einfach durch Methyl, Ethyl, Propyl, Isopropyl, Trifluormethyl, Methoxy, Ethoxy, Propoxy, Butoxy oder Cyclopropyl-methoxy substituiert ist,
- A, B: und das Kohlenstoffatom, an das sie gebunden sind, stehen ganz besonders bevorzugt für C₆-Cycloalkyl, welches gegebenenfalls durch mit zwei nicht direkt benachbarten Sauerstoffatomen enthaltende C₂-C₃- Alkylendioxyl-Gruppe substituiert ist,
- A, B: und das Kohlenstoffatom, an das sie gebunden sind, stehen ganz besonders bevorzugt für C₅-C₆-Cycloalkyl oder C₅-C₆-Cycloalkenyl, worin zwei Substituenten gemeinsam mit den Kohlenstoffatomen, an die sie gebunden sind, für C₂-C₄-Alkandiyl oder C₂-C₄-Alkendiyl oder Butadiendiyl stehen, mit der Maßgabe, dass dann Q³ ganz besonders bevorzugt für Wasserstoff steht,
- D: steht ganz besonders bevorzugt für Wasserstoff, für jeweils gegebenenfalls einfach bis dreifach durch Fluor substituiertes C₁-C₄-Alkyl, C₃-C₄-Alkenyl, C₁-C₄-Alkoxy-C₂-C₃-alkyl, für Cyclopropyl, Cyclopentyl oder Cyclohexyl
oder
- A und D: stehen gemeinsam ganz besonders bevorzugt für gegebenenfalls einfach durch Methyl oder Methoxy substituiertes C₃-C₅-Alkandiyl, worin gegebenenfalls ein Kohlenstoffatom durch Sauerstoff oder Schwefel ersetzt ist oder für die Gruppe AD-1.
- X: steht hervorgehoben für Methoxy, Ethoxy, n-Propoxy, iso-Propoxy, n-Butoxy, iso-Butoxy, sec-Butoxy, Methoxy-ethyloxy, Ethoxy-ethyloxy oder Cyclopropylmethoxy,
- W: steht hervorgehoben für Methyl oder Ethyl,
- Y: steht hervorgehoben für Chlor,
- G: steht hervorgehoben für Lithium, Natrium, Kalium, Calcium, Magnesium oder Brom;
- m: steht hervorgehoben für die Zahl 1,
- n: steht hervorgehoben für die Zahl 1 oder 2,
- A: steht hervorgehoben für Wasserstoff, Methyl, Ethyl, Isopropyl, n-Propyl, Isobutyl, n-Butyl, t-Butyl, s-Butyl oder Cyclopropyl
- B: steht hervorgehoben für Wasserstoff oder Methyl,
- A, B: und das Kohlenstoffatom an das sie gebunden sind, stehen hervorgehoben für gesättigtes C₅-C₆-Cycloalkyl, in welchem gegebenenfalls ein Ringglied durch Sauerstoff ersetzt ist und welches gegebenenfalls einfach durch Methyl, Methoxy, Ethoxy, n-Propoxy, n-Butoxy oder Trifluormethyl (insbesondere durch Methyl oder Methoxy), substituiert ist,
- D: steht hervorgehoben für Wasserstoff, Methyl, Ethyl, Isopropyl, Cyclopropyl oder Cyclohexyl,
oder

A und D stehen gemeinsam hervorgehoben für C₃-C₅-Alkandiyl oder für die Gruppe AD-1.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß hervorgehoben werden die Verbindungen der Formel (I), in welchen eine Kombination der vorstehend als hervorgehoben aufgeführten Bedeutungen vorliegt.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

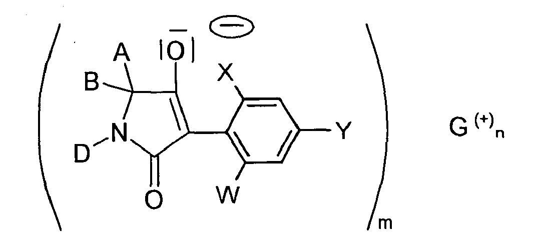

**Tabelle 1: W = OCH₃, X = CH₃, Y = Cl; G⁽⁺⁾n = Na⁺; m = 1**

| A | B | D |
|---|---|---|
| CH₃ | H | H |
| C₂H₅ | H | H |
| C₃H₇ | H | H |
| i-C₃H₇ | H | H |
| C₄H₉ | H | H |
| i-C₄H₉ | H | H |
| s-C₄H₉ | H | H |
| t-C₄H₉ | H | H |
| CH₃ | CH₃ | H |
| C₂H₅ | CH₃ | H |
| C₃H₇ | CH₃ | H |
| i-C₃H₇ | CH₃ | H |
| C₄H₉ | CH₃ | H |
| i-C₄H₉ | C^{H}3 | H |
| s-C₄H₉ | CH₃ | H |
| t-C₄H₉ | C^{H}3 | H |
| C₂H₅ | C₂H₅ | H |
| C₃H₇ | C₃H₇ | H |
| -(CH₂)₂- | | H |
| -(CH₂)₄- | | H |
| -(CH₂)₅- | | H |
| -(CH₂)₆- | | H |
| -(CH₂)₇- | | H |
| -(CH₂)₂-O-(CH₂)₂- | | H |
| -CH₂-O-(CH₂)₃- | | H |
| -(CH₂)₂-S-(CH₂)₂- | | H |
| -CH₂-CHCH₃-(CH₂)₃- | | H |
| -(CH₂)₂-CHCH₃-(CH₂)₂- | | H |
| -(CH₂)₂-CHC₂H₅-(CH₂)₂- | | H |
| -(CH₂)₂-CHC₃H₇-(CH₂)₂- | | H |
| -(CH₂)₂-CHi-C₃H₇-(CH₂)₂- | | H |
| -(CH₂)₂-CHOCH₃-(CH₂)₂- | | H |
| -(CH₂)₂-CHOC₂H₅-(CH₂)₂- | | H |
| -(CH₂)₂-CHOC₃H₇-(CH₂)₂- | | H |
| -(CH₂)₂-CHO-i-C₃H₇-(CH₂)₂- | | H |
| -(CH₂)₂-C(CH₃)₂-(CH₂)₂- | | H |
| -CH₂-(CHCH₃)₂-(CH₂)₂- | | H |
| 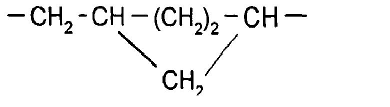 | | H |
| 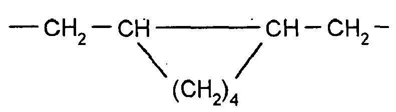 | | H |
| 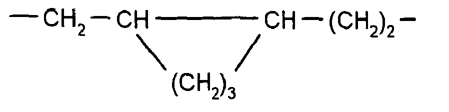 | | H |

| A | B | D |
|---|---|---|
| 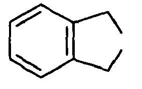 | | H |
| 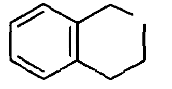 | | H |

| A | D | B |
|---|---|---|
| -(CH₂)₃- | | H |
| -(CH₂)₄- | | H |
| -CH₂-CHCH₃-CH₂- | | H |
| -CH₂-CH₂-CHCH₃- | | H |
| -CH₂-CHCH₃-CHCH₃- | | H |
| -CH₂-CH(OCH₃)-CH₂- | | H |
| -CH₂-CH=CH-CH₂- | | H |
| -CH₂-CH-CH-CH₂- | | H |
| -CH₂-S-CH₂- | | H |
| -CH₂-S-(CH₂)₂- | | H |
| -(CH₂)₂-S-CH₂- | | H |
| 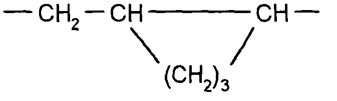 | | H |
| H | CH₃ | H |
| H | C₂H₅ | H |
| H | C₃H₇ | H |
| H | i-C₃H₇ | H |
| H | 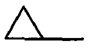 | H |
| H | 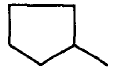 | H |

| A | D | B |
|---|---|---|
| H | 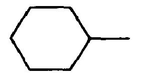 | H |
| CH₃ | CH₃ | H |
| CH₃ | C₂H₅ | H |
| CH₃ | C₃H₇ | H |
| CH₃ | i-C₃H₇ | H |
| CH₃ | 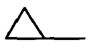 | H |
| CH₃ | 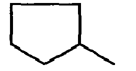 | H |
| CH₃ | 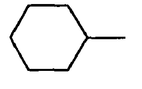 | H |
| 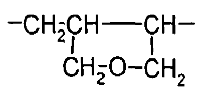 | | H |
| C₂H₅ | CH₃ | H |
| C₂H₅ | C₂H₅ | H |

### Tabelle 2: A, B und D wie in Tabelle 1 angegeben

W = OCH₃; X = CH₃; Y = Br; G⁽⁺⁾n = Na⁺; m = 1

### Tabelle 3: A, B und D wie in Tabelle 1 angegeben

W = OCH₃; X = C₂H₅; Y = Cl; G⁽⁺⁾n = Na⁺; m = 1

### Tabelle 4: A, B und D wie in Tabelle 1 angegeben

W = OCH₃; X = C₂H₅; Y = Br; G⁽⁺⁾ₙ = Na⁺; m = 1

### Tabelle 5: A, B und D wie in Tabelle 1 angegeben

W = OC₂H₅; X = CH₃; Y =Cl; G(+)ₙ = Na⁺; m = 1

### Tabelle 6: A, B und D wie in Tabelle 1 angegeben

W = OC₂H₅; X = C₂H₅; Y = Cl; G⁽⁺⁾ₙ = Na⁺; m = 1

Bevorzugte Bedeutungen der oben in Zusammenhang mit den die Kulturpflanzen-Verträglichkeit verbessernden Verbindungen ("Herbizid-Safenem") der Formeln (IIa), (IIb), (IIc), (IId) und (IIe) aufgeführten Gruppen werden im Folgenden definiert.
- n: steht bevorzugt für die Zahlen 0, 1, 2, 3 oder 4.
- A¹: steht bevorzugt für eine der nachstend skizzierten divalenten heterocyclischen Gruppierungen

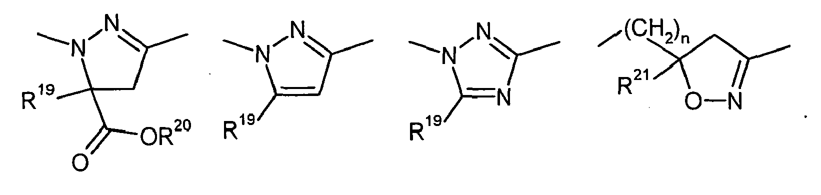

steht bevorzugt für jeweils gegebenenfalls durch Methyl, Ethyl, Methoxycarbonyl oder Ethoxy-carbonyl substituiertes Methylen oder Ethylen.
- R¹⁴: steht bevorzugt für Hydroxy, Mercapto, Amino, Methoxy, Ethoxy, n- oder i-Propoxy, n-, i-, s-oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁵: steht bevorzugt für Hydroxy, Mercapto, Amino, Methoxy, Ethoxy, n- oder i-Propoxy, n-, i-, s-oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁶: steht bevorzugt für jeweils gegebenenfalls durch Fluor, Chlor und/oder Brom substituiertes Methyl, Ethyl, n- oder i-Propyl.
- R¹⁷: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanyhnethyl, Furyl, Furylmethyl, Thienyl, Thiazolyl, Piperidinyl, oder gegebenenfalls durch Fluor, Chlor, Methyl, Ethyl, n-oder i-Propyl, n-, i-, s- oder t-Butyl substituiertes Phenyl.
- R¹⁸: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n-oder i-Propyl, n-, i-, s- oder t-Butyl substituiertes Phenyl, oder zusammen mit R¹⁷ für einen der Reste -CH₂-O-CH₂-CH₂- und -CH₂-CH₂-O-CH₂-CH₂-, die gegebenenfalls substituiert sind durch Methyl, Ethyl, Furyl, Phenyl, einen annellierten Benzolring oder durch zwei Substituenten, die gemeinsam mit dem C-Atom, an das sie gebunden sind, einen 5- oder 6-gliedrigen Carbocyclus bilden.
- R¹⁹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, Cyclopropyl, Cyclobutyl, Cyclopentyl, Cyclohexyl oder Phenyl.
- R²⁰: steht bevorzugt für Wasserstoff, gegebenenfalls durch Hydroxy, Cyano, Fluor, Chlor, Methoxy, Ethoxy, n- oder i-Propoxy substituiertes Methyl, Ethyl, n- oder i-Propyl, n-, i-, s- oder t-Butyl.
- R²¹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, n-, i-, s- oder t-Butyl, Cyclopropyl, Cyclobutyl, Cyclopentyl, Cyclohexyl oder Phenyl.
- X¹: steht bevorzugt für Nitro, Cyano, Fluor, Chlor, Brom, Methyl, Ethyl, n- oder i-Propyl, n-, i-, s-oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- X²: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- X³: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- R²²: steht bevorzugt für Wasserstoff, Methyl, Ethyl, n- oder i-Propyl.
- R²³: steht bevorzugt für Wasserstoff, Methyl, Ethyl, n- oder i-Propyl.
- R²⁴: steht bevorzugt für Wasserstoff, jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio,, Methylamino, Ethylamino, n- oder i-Propylamino, n-, i-, s-oder t-Butylamino, Dimethylamino oder Diethylamino, oder jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino oder Cyclohexylamino.
- R²⁵: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, oder jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl.
- R²⁶: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl, oder gegebenenfalls durch Nitro, Cyano, Fluor, Chlor, Brom, Methyl, Ethyl, n- oder i-Propyl, n-, i-, s-oder t-Butyl, Trifluormethyl, Methoxy, Ethoxy, n- oder i-Propoxy, Difluormethoxy oder Trifluormethoxy substituiertes Phenyl, oder zusammen mit R²⁵ für jeweils gegebenenfalls durch Methyl oder Ethyl substituiertes Butan-1,4-diyl (Trimethylen), Pentan-1,5-diyl, 1-Oxa-butan-1,4-diyl oder 3-Oxa-pentan-1,5-diyl.
- X⁴: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.
- X⁵: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.

Beispiele für die als erfindungsgemäße Herbizid-Safener ganz besonders bevorzugten Verbindungen der Formel (IIa) sind in der nachstehenden Tabelle aufgeführt.

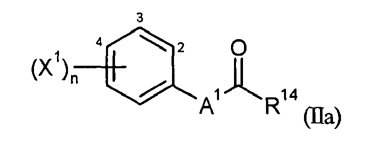

**Tabelle Beispiele für die Verbindungen der Formel (IIa)**

| **Beispiel- Nr.** | **(Positionen) (X¹)ₙ** | A¹ | **R¹⁴** |
|---|---|---|---|
| IIa-1 | (2) Cl, (4) Cl | 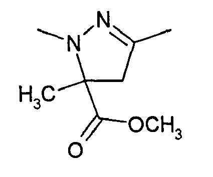 | OCH₃ |
| IIa-2 | (2) Cl, (4) Cl | 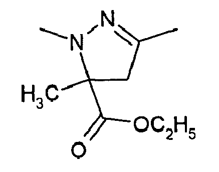 | OCH₃ |
| IIa-3 | (2) Cl, (4) Cl | 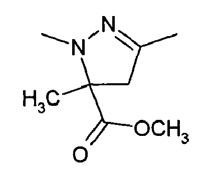 | OC₂H₅ |
| IIa-4 | (2) Cl, (4) Cl | 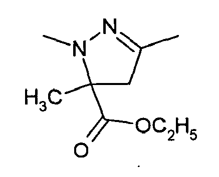 | OC₂H₅ |
| IIa-5 | (2) Cl | 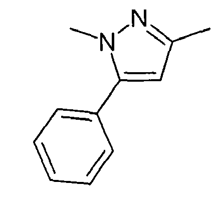 | OCH₃ |
| Ma-6 | (2) C1, (4) C1 | 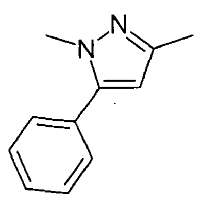 | OCH₃ |
| IIa-7 | (2) F | 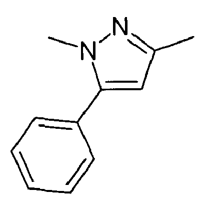 | OCH₃ |
| IIa-8 | (2) F | 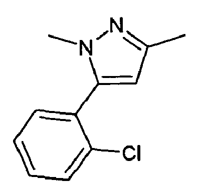 | OCH₃ |
| IIa-9 | (2)C1,(4)C1 | 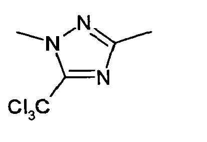 | OC₂H₅ |
| IIa-10 | (2) Cl, (4) CF₃ | 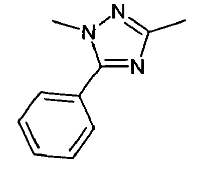 | OCH₃ |
| IIa-11 | (2) Cl | 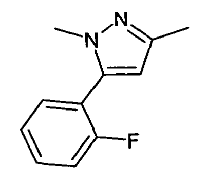 | OCH₃ |
| IIa-12 | - | 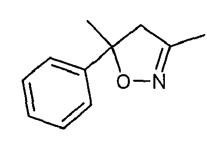 | OC₂H₅ |
| IIa-13 | (2) Cl, (4)Cl | 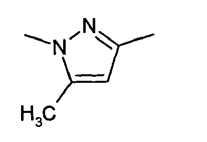 | OC₂H₅ |
| IIa-14 | (2) Cl, (4) Cl | 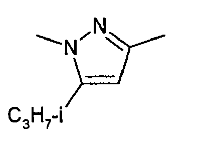 | OC₂H₅ |
| IIa-15 | (2) Cl, (4) Cl | 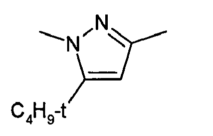 | OC₂H₅ |
| IIa-16 | (2) Cl, (4) Cl | 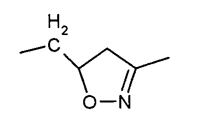 | OC₂H₅ |
| IIa-17 | (2) Cl, (4) Cl | 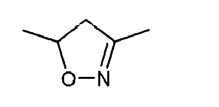 | OC₂H₅ |
| IIa-18 | - | 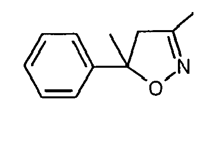 | OH |

Beispiele für die als erfindungsgemäße Herbizid-Safener ganz besonders bevorzugten Verbindungen der Formel (IIb) sind in der nachstehenden Tabelle aufgeführt.

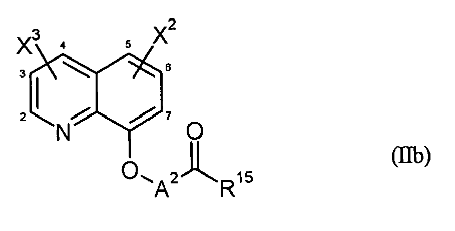

**Tabelle Beispiele für die Verbindungen der Formel (IIb)**

| **Beispiel- Nr.** | **(Position) X²** | **(Position) X³** | **A²** | **R¹⁵** |
|---|---|---|---|---|
| IIb-1 | (5) Cl | - | CH₂ | OH |
| IIb-2 | (5) Cl | - | CH₂ | OCH₃ |
| IIb-3 | (5) C1 | - | CH₂ | OC₂H₅ |
| IIb-4 | (5) C1 | - | CH₂ | OC₃H₇-n |
| IIb-5 | (5) C1 | - | CH₂ | OC₃H₇-i |
| IIb-6 | (5) Cl | - | CH₂ | OC₄H₉-n |
| IIb-7 | (5) C1 | - | CH₂ | OCH(CH₃)C₅H₁₁-n |
| IIb-8 | (5) Cl | (2) F | CH₂ | OH |
| IIb-9 | (5) Cl | (2) Cl | CH₂ | OH |
| IIb-10 | (5) Cl | - | CH₂ | OCH₂CH=CH₂ |
| IIb-11 | (5) Cl | - | CH₂ | OC₄H₉-i |
| IIb-12 | (5) Cl | - | CH₂ | 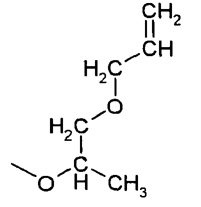 |
| IIb-13 | (5) Cl | - | 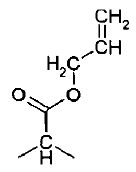 | OCH₂CH=CH₂ |
| IIb-14 | (5) Cl | - | 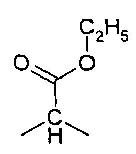 | OC₂H₅ |
| IIb-15 | (5) Cl | - | 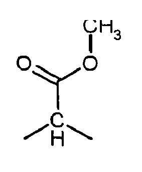 | OCH₃ |

Beispiele für die als erfindungsgemäße Herbizid-Safener ganz besonders bevorzugten Verbindungen der Formel (IIc) sind in der nachstehenden Tabelle aufgeführt.

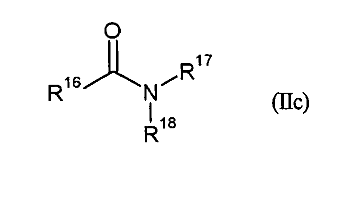

**Tabelle Beispiele für die Verbindungen der Formel (IIc)**

| **Beispiel- Nr.** | **R¹⁶** | **N(R¹⁷,R¹⁸)** |
|---|---|---|
| IIc-1 | CHCl₂ | N(CH₂CH=CH₂)₂ |
| IIc-2 | CHCl₂ | 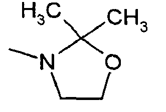 |
| IIc-3 | CHCl₂ | 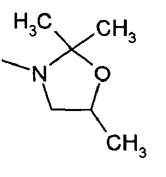 |
| IIc-4 | CHCl₂ | 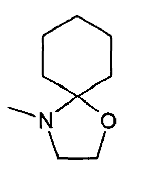 |
| IIc-5 | CHCl₂ | 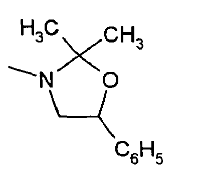 |
| IIc-6 | CHCl₂ | 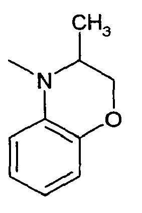 |
| IIc-7 | CHCl₂ | 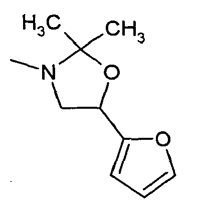 |

Beispiele für die als erfindungsgemäße Herbizid-Safener ganz besonders bevorzugten Verbindungen der Formel (IId) sind in der nachstehenden Tabelle aufgeführt.

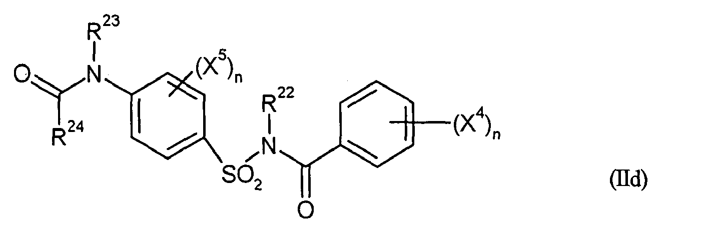

**Tabelle Beispiele für die Verbindungen der Formel (IId)**

| **Beispiel- Nr.** | **R²²** | **R²³** | **R²⁴** | **(Positionen) (X⁴)ₙ** | **(Positionen) (X⁵)ₙ** |
|---|---|---|---|---|---|
| IId-1 | H | H | CH₃ | (2) OCH₃ | - |
| IId-2 | H | H | C₂H₅ | (2) OCH₃ | - |
| IId-3 | H | H | C₃H₇-n | (2) OCH₃ | - |
| IId-4 | H | H | C₃H₇-i | (2) OCH₃ | - |
| IId-5 | H | H | 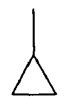 | (2) OCH₃ | - |
| IId-6 | H | H | CH₃ | (2) OCH₃ (5) CH₃ | - |
| IId-7 | H | H | C₂H₅ | (2) OCH₃ (5) CH₃ | - |
| IId-8 | H | H | C₃H₇-n | (2) OCH₃ (5) CH₃ | - |
| IId-9 | H | H | C₃H₇-i | (2) OCH₃ (5) CH₃ | - |
| IId-10 | H | H | 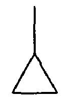 | (2) OCH₃ (5) CH₃ | - |
| IId-11 | H | H | OCH₃ | (2) OCH₃ (5) CH₃ | - |
| nd-12 | H | H | OC₂H₅ | (2) OCH₃ (5) CH₃ | - |
| IId-13 | H | H | OC₃H₇-i | (2) OCH₃ (5) CH₃ | - |
| IId-14 | H | H | SCH₃ | (2)OCH₃ (5) CH₃ | - |
| IId-15 | H | H | SC₂H₅ | (2) OCH₃ (5) CH₃ | - |
| IId-16 | H | H | SC₃H₇-i | (2) OCH₃ (5)CH₃ | - |
| IId-17 | H | H | NHCH₃ | (2) OCH₃ (5) CH₃ | - |
| IId-18 | H | H | NHC₂H₅ | (2) OCH₃ (5) CH₃ | - |
| IId-19 | H | H | NHC₃H₇-i | (2) OCH₃ (5) CH₃ | - |
| IId-20 | H | H | 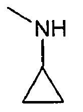 | (2) OCH₃ (5) CH₃ | - |
| IId-21 | H | H | NHCH₃ | (2) OCH₃ | - |
| IId-22 | H | H | NHC₃H₇-i | (2) OCH₃ | - |
| IId-23 | H | H | N(CH₃)₂ | (2) OCH₃ | - |
| IId-24 | H | H | N(CH₃)₂ | (3) CH₃ (4) CH₃ | - |
| IId-25 | H | H | CH₂-O-CH₃ | (2) OCH₃ | - |

Beispiele für die als erfindungsgemäße Herbizid-Safener ganz besonders bevorzugten Verbindungen der Formel (IIe) sind in der nachstehenden Tabelle aufgeführt.

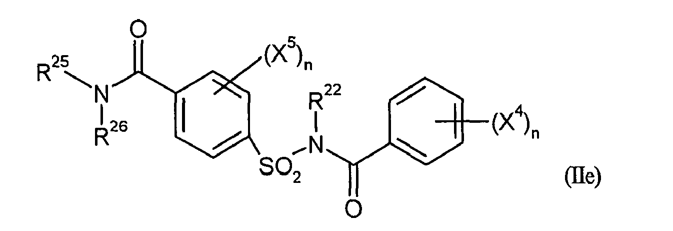

**Tabelle Beispiele für die Verbindungen der Formel (IIe)**

| **Beispiel- Nr.** | R²² | R²⁵ | **R²⁶** | (Positionen) **(X⁴)ₙ** | (Positionen) **(X⁵)ₙ** |
|---|---|---|---|---|---|
| IIe-1 | H | H | CH₃ | (2) OCH₃ | - |
| IIe-2 | H | H | C₂H₅ | (2) OCH₃ | - |
| IIe-3 | H | H | C₃H₇-n | (2) OCH₃ | - |
| IIe-4 | H | H | C₃H₇-i | (2) OCH₃ | - |
| IIe-5 | H | H | 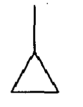 | (2) OCH₃ | - |
| IIe-6 | H | CH₃ | CH₃ | (2) OCH₃ | - |
| IIe-7 | H | H | CH₃ | (2) OCH₃ (5) CH₃ | - |
| IIe-8 | H | H | C₂H₅ | (2) OCH₃ (5) CH₃ | - |
| IIe-9 | H | H | C₃H₇-n | (2) OCH₃ (5) CH₃ | - |
| IIe-10 | H | H | C₃H₇-i | (2) OCH₃ (5) CH₃ | - |
| IIe-11 | H | H | 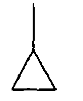 | (2) OCH₃ (5) CH₃ | - |
| IIe-12 | H | CH₃ | CH₃ | (2) OCH₃ (5) CH₃ | - |

Als die die Kulturpflanzen-Verträglichkeit verbessernde Verbindung [Komponente (b')] sind Cloquintocet-mexyl, Fenchlorazol-ethyl, Isoxaifen-ethyl, Mefenpyr-diethyl, Furilazole, Fenclorim, Cumyluron, Dymron, Dimepiperate und die Verbindungen IIe-5 und IIe-11 am meisten bevorzugt, wobei Cloquintocet-mexyl und Mefenpyr-diethyl besonders hervorgehoben seien.

Die als Safener erfindungsgemäß zu verwendenden Verbindungen der allgemeinen Formel (IIa) sind bekannt und/oder können nach an sich bekannten Verfahren hergestellt werden (vgl. WO-A-91/07874, WO-A-95/07897).

Die als Safener erfindungsgemäß zu verwendenden Verbindungen der allgemeinen Formel (IIb) sind bekannt und/oder können nach an sich bekannten Verfahren hergestellt werden (vgl. EP-A-191736).

Die als Safener erfindungsgemäß zu verwendenden Verbindungen der allgemeinen Formel (IIc) sind bekannt und/oder können nach an sich bekannten Verfahren hergestellt werden (vgl. DE-A-2218097, DE-A-2350547).

Die als Safener erfindungsgemäß zu verwendenden Verbindungen der allgemeinen Formel (IId) sind bekannt und/oder können nach an sich bekannten Verfahren hergestellt werden (vgl. DE-A-19621522/USA-6235680).

Die als Safener erfindungsgemäß zu verwendenden Verbindungen der allgemeinen Formel (IIe) sind bekannt und können nach an sich bekannten Verfahren hergestellt werden (vgl. WO-A-99/66795/US-A-6251827).

Beispiele für die erfindungsgemäßen selektiv herbiziden Kombinationen aus jeweils einem Wirkstoff der Formel (I) und jeweils einem der oben definierten Safener sind in der nachstehenden Tabelle aufgeführt.

**Tabelle Beispiele für die erfindungsgemäßen Kombinationen**

| **Wirkstoffe der Formel (I)** | **Safener** |
|---|---|
| A.1 | Cloquintocet-mexyl |
| A.1 | Fenchlorazole-ethyl |
| A.1 | Isoxadifen-ethyl |
| A.1 | Mefenpyr-diethyl |
| A.1 | Furilazole |
| A.1 | Fenclorim |
| A.1 | Cumyluron |
| A.1 | Daimuron /Dymron |
| A.1 | Dimepiperate |
| A.1 | IIe-11 |
| A.1 | IIe-5 |
| A.2 | Cloquintocet-mexyl |
| A.2 | Fenchlorazole-ethyl |
| A.2 | Isoxadifen-ethyl |
| A.2 | Mefenpyr-diethyl |
| A.2 | Furilazole |
| A.2 | Fenclorim |
| A.2 | Cumyluron |
| A.2 | Daimuron /Dymron |
| A.2 | Dimepiperate |
| A.2 | IIe-11 |
| A.2 | IIe-5 |
| A.3 | Cloquintocet-mexyl |
| A.3 | Fenchlolorazole-ethyl |
| A.3 | Isoxadifen-ethyl |
| A.3 | Mefenpyr-diethyl |
| A.3 | Furilazole |
| A.3 | Fenclorim |
| A.3 | Cumyluron |
| A.3 | Daimuron /Dymron |
| A.3 | Dimepiperate |
| A.3 | IIe-5 |
| A.3 | IIe-11 |
| A.4 | Cloquintocet-mexyl |
| A.4 | Fenchlorazole-ethyl |
| A.4 | Isoxadifen-ethyl |
| A.4 | Mefenpyr-diethyl |
| A.4 | Furilazole |
| A.4 | Fenclorim |
| A.4 | Cumyluron |
| A.4 | Daimuron /Dymron |
| A.4 | Dimepiperate |
| A.4 | IIe-11 |
| A.4 | IIe-5 |
| A.5 | Cloquintocet-mexyl |
| A.5 | Fenchiorazole-ethyl |
| A.5 | Isoxadifen-ethyl |
| A.5 | Mefenpyr-diethyl |
| A.5 | Furilazole |
| A.5 | Fenclorim |
| A.5 | Cumyluron |
| A.5 | Daimuron /Dymron |
| A.5 | Dimepiperate |
| A.5 | IIe-5 |
| A.5 | IIe-11 |
| A.6 | Cloquintocet-mexyl |
| A.6 | Fenchlorazole-ethyl |
| A.6 | Isoxadifen-ethyl |
| A.6 | Mefenpyr-diethyl |
| A.6 | Furilazole |
| A.6 | Fenclorim |
| A.6 | Cumyluron |
| A.6 | Daimuron /Dymron |
| A.6 | Dimepiperate |
| A.6 | IIe-5 |
| A.6 | IIe-11 |
| A.7 | Cloquintocet-mexyl |
| A.7 | Fenchlorazole-ethyl |
| A.7 | Isoxadifen-ethyl |
| A.7 | Mefenpyr-diethyl |
| A.7 | Furilazole |
| A.7 | Fenclorim |
| A.7 | Cumyluron |
| A.7 | Daimuron/Dymron |
| A.7 | Dimepiperate |
| A.7 | IIe-5 |
| A.7 | IIe-11 |
| A.8 | Cloquintocet-mexyl |
| A.8 | Fenchlorazole-ethyl |
| A.8 | Isoxadifen-ethyl |
| A.8 | Mefenpyr-diethyl |
| A.8 | Furilazole |
| A.8 | Fenclorim |
| A.8 | Cumyluron |
| A.8 | Daimuron /Dymron |
| A.8 | Dimepiperate |
| A.8 | IIe-5 |
| A.8 | IIe-11 |
| A.9 | Cloquintocet-mexyl |
| A.9 | Fenchlorazole-ethyl |
| A.9 | Isoxadifen-ethyl |
| A.9 | Mefenpyr-diethyl |
| A.9 | Furilazole |
| A.9 | Fenclorim |
| A.9 | Cumyluron |
| A.9 | Daimuron/Dymron |
| A.9 | Dimepiperate |
| A.9 | IIe-5 |
| A.9 | IIe-11 |
| A.10 | Cloquintocet-mexyl |
| A.10 | Fenchlorazole-ethyl |
| A.10 | Isoxadifen-ethyl |
| A.10 | Mefenpyr-diethyl |
| A.10 | Furilazole |
| A.10 | Fenclorim |
| A.10 | Cumyluron |
| A.10 | Daimuron /Dymron |
| A.10 | Dimepiperate |
| A.10 | IIe-5 |
| A.10 | IIe-11 |
| A.11 | Cloquintocet-mexyl |
| A.11 | Fenchlorazole-ethyl |
| A.11 | Isoxadifen-ethyl |
| A.11 | Mefenpyr-diethyl |
| A.11 | Furilazole |
| A.11 | Fenclorim |
| A.11 | Cumyluron |
| A.11 | Daimuron/Dymron |
| A.11 | Dimepiperate |
| A.11 | IIe-5 |
| A.11 | IIe-11 |
| A.12 | Cloquintocet-mexyl |
| A.12 | Fenchlorazole-ethyl |
| A.12 | Isoxadifen-ethyl |
| A.12 | Mefenpyr-diethyl |
| A.12 | Furilazole |
| A.12 | Fenclorim |
| A.12 | Cumyluron |
| A.12 | Daimuron/Dymron |
| A.12 | Dimepiperate |
| A.12 | IIe-5 |
| A.12 | IIe-11 |
| A.13 | Cloquintocet-mexyl |
| A.13 | Fenchlorazole-ethyl |
| A.13 | Isoxadifen-ethyl |
| A.13 | Mefenpyr-diethyl |
| A.13 | Furilazole |
| A.13 | Fenclorim |
| A.13 | Cumyluron |
| A.13 | Daimuron /Dymron |
| A.13 | Dimepiperate |
| A.13 | IIe-5 |
| A.13 | IIe-11 |
| A.14 | Cloquintocet-mexyl |
| A.14 | Fenchlorazole-ethyl |
| A.14 | Isoxadifen-ethyl |
| A.14 | Mefenpyr-diethyl |
| A.14 | Furilazole |
| A.14 | Fenclorim |
| A.14 - | Cumyluron |
| A.14 | Daimuron/Dymron |
| A.14 | Dimepiperate |
| A.14 | IIe-5 |
| A.14 | IIe-11 |
| A.15 | Cloquintocet-mexyl |
| A.15 | Fenchlorazole-ethyl |
| A.15 | Isoxadifen-ethyl |
| A15 | Mefenpyr-diethyl |
| A.15 | Furilazole |
| A.15 | Fenclorim |
| A.15 | Cumyluron |
| A.15 | Daimuron/Dymron |
| A.15 | Dimepiperate |
| A.15 | IIe-5 |
| A.15 | IIe-11 |
| A.16 | Cloquintocet-mexyl |
| A.16 | Fenchlorazole-ethyl |
| A.16 | Isoxadifen-ethyl |
| A.16 | Mefenpyr-diethyl |
| A.16 | Furilazole |
| A.16 | Fenclorim |
| A.16 | Cumyluron |
| A.16 | Daimuron /Dymron |
| A.16 | Dimepiperate |
| A.16 | IIe-5 |
| A.16 | IIe-11 |
| A.17 | Cloquintocet-mexyl |
| A.17 | Fenchlorazole-ethyl |
| A.17 | Isoxadifen-ethyl |
| A.17 | Mefenpyr-diethyl |
| A.17 | Furilazole |
| A.17 | Fenclorim |
| A.17 | Cumyluron |
| A.17 | Daimuron/Dymron |
| A.17 | Dimepiperate |
| A.17 | IIe-5 |
| A.17 | IIe-11 |
| A.18 | Cloquintocet-mexyl |
| A.18 | Fenchlorazole-ethyl |
| A.18 | Isoxadifen-ethyl |
| A.18 | Mefenpyr-diethyl |
| A.18 | Furilazole |
| A.18 | Fenclorim |
| A.18 | Cumyluron |
| A.18 | Daimuron/Dymron |
| A.18 | Dimepiperate |
| A.18 | IIe-5 |
| A.18 | IIe-11 |
| A.19 | Cloquintocet-mexyl |
| A.19 | Fenchlorazole-ethyl |
| A.19 | Isoxadifen-ethyl |
| A.19 | Mefenpyr-diethyl |
| A.19 | Furilazole |
| A.19 | Fenclorim |
| A.19 | Cumyluron |
| A.19 | Daimuron/Dymron |
| A.19 | Dimepiperate |
| A.19 | IIe-5 |
| A.19 | IIe-11 |
| A.20 | Cloquintocet-mexyl |
| A.20 | Fenchlorazole-ethyl |
| A.20 | Isoxadifen-ethyl |
| A.20 | Mefenpyr-diethyl |
| A.20 | Furilazole |
| A.20 | Fenclorim |
| A.20 | Cumyluron |
| A.20 | Daimuron/Dymron |
| A.20 | Dimepiperate |
| A.20 | IIe-5 |
| A.20 | IIe-11 |
| A.21 | Cloquintocet-mexyl |
| A.21 | Fenchlorazole-ethyl |
| A.21 | Isoxadifen-ethyl |
| A.21 | Mefenpyr-diethyl |
| A.21 | Furilazole |
| A.21 | Fenclorim |
| A.21 | Cumyluron |
| A.21 | Daimuron /Dymron |
| A.21 | Dimepiperate |
| A.21 | IIe-5 |
| A.21 | IIe-11 |

Es wurde nun überraschend gefunden, dass die oben definierten Wirkstoffkombinationen aus 3-(2-Alkoxyphenyl)-substituierten Tetramate der allgemeinen Formel (I) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von 3-(2-Alkoxy-phenyl)-substituierten Tetramate auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

Alle in den erfindungsgemäßen Mitteln enthaltenen Wirkstoffe der Formel (I) können nach im Stand der Technik beschriebenen Verfahren (siehe oben genannte Referenzen) hergestellt werden. Ihre Wirkung ist gut, jedoch insbesondere bei niedrigen Aufwandmengen und Konzentrationen nicht immer voll zufriedenstellend. Weiterhin ist die Pflanzenverträglichkeit dieser Verbindungen nicht immer ausreichend. Es besteht deshalb ein Bedarf für eine Wirkungssteigerung und/oder Verbesserung der Pflanzenverträglichkeit gegenüber Kulturpflanzen der die Verbindungen enthaltenden Pflanzenschutzmittel.

In der Literatur wurde bereits beschrieben, dass sich die Wirkung verschiedener Wirkstoffe durch Zugabe von Ammoniumsalzen steigern lässt. Dabei handelt es sich jedoch um als Detergens wirkende Salze (z.B. WO 95/017817) bzw. Salze mit längeren Alkyl- und / oder Arylsubstituenten, die permeabilisierend wirken oder die Löslichkeit des Wirkstoffs erhöhen (z.B. EP-A 0 453 086, EP-A 0 664 081, FR-A 2 600 494, US 4 844 734, US 5 462 912, US 5 538 937, US-A 03/0224939, US-A 05/0009880, US-A 05/0096386). Weiterhin beschreibt der Stand der Technik die Wirkung nur für bestimmte Wirkstoffe und / oder bestimmte Anwendungen der entsprechenden Mittel. Eine Wirkungssteigerung z.B. durch Ammoniumsulfat ist beispielsweise für die Herbizide Glyphosat und Phosphinothricin beschrieben (US 6 645 914, EP-A2 0 036 106).

Auch der Einsatz von Ammoniumsulfat als Forrnulierhilfsrniael ist für bestimmte Wirkstoffe und Anwendungen beschrieben (WO 92/16108), es dient dort aber zur Stabilisierung der Formulierung, nicht zur Wirkungssteigerung.

Herbizide bzw. insektizide Zusammensetzungen von cyclischen Ketoenolen mit Ammonium- oder Phosphoniumsalzen zur Wirkstoffsteigerung sind beschrieben in DE-05059469 und DE-05059471 (noch nicht veröffentlicht).

Es wurde nun völlig überraschend gefunden, dass sich die Wirkung von Herbiziden und/oder Akariziden und/oder Insektiziden aus der Klasse der 3-(2-Alkoxy-phenyl)-substituierten Tetramate durch den Zusatz von Ammonium- oder Phosphoniumsalzen zur Anwendungslösung oder durch den Einbau dieser Salze in eine Formulierung enthaltend 3-(2-Alkoxy-phenyl)-substituierten Tetramate, deutlich steigern lässt. Gegenstand der vorliegenden Erfindung ist also die Verwendung von Ammonium- oder Phosphoniumsalzen zur Wirkungssteigerung von Pflanzenschutzmitteln, die insektizid und/oder akarizid und/oder herbizid wirksame 3-(2-Alkoxy-phenyl)-substituierte Tetramate als Wirkstoff enthalten. Gegenstand der Erfindung sind ebenfalls Mittel, die insektizid und/oder akarizid und/oder herbizid wirksame 3-(2-Alkoxy-phenyl)-substituierte Tetramate und die Wirkung steigernde Ammonium- oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von unerwünschtem Pflanzenwuchs.

Die Wirkstoffe können in den erfindungsgemäßen Zusammensetzungen in einem breiten Konzentrationsbereich eingesetzt werden. Die Konzentration der Wirkstoffe in der Formulierung beträgt dabei üblicherweise 0,1 - 50 Gew.-%.

Ammonium- und Phosphoniumsalze, die erfindungsgemäß die Wirkung von Pflanzenschutzmitteln enthaltend 3-(2-Alkoxy-phenyl)-substituierte Tetramate steigern, werden durch Formel (II') definiert 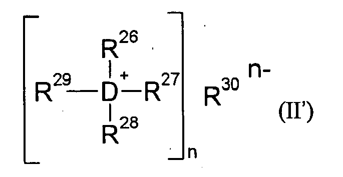 in welcher
- D: für Stickstoff oder Phosphor steht,
- D: bevorzugt für Stickstoff steht,
- R²⁶, R²⁷, R²⁸ und R²⁹: unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
- R²⁶, R²⁷, R²⁸ und R²⁹: bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
- R²⁶, R²⁷, R²⁸ und R²⁹: besonders bevorzugt unabhängig voneinander für Wasserstoff, Methyl, Ethyl, n-Propyl, i-Propyl, n-Butyl, i-Butyl, s-Butyl oder t-Butyl stehen,
- R²⁶, R²⁷, R²⁸ und R²⁹: ganz besonders bevorzugt für Wasserstoff stehen,
- R²⁶, R²⁷, R²⁸ und R²⁹: weiterhin ganz besonders bevorzugt gleichzeitig für Methyl oder gleichzeitig für Ethyl stehen,
- n: für 1, 2, 3 oder 4 steht,
- n: bevorzugt für 1 oder 2 steht,
- R³⁰: für ein anorganisches oder organisches Anion steht,
- R³⁰: bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat, Citrat oder Oxalat steht,
- R³⁰: weiterhin bevorzugt für Carbonat, Pentaborat, Sulfit, Benzoat, Hydrogenoxalat, Hydrogencitrat, Methylsulfat oder Tetrafluoroborat steht,
- R³⁰: besonders bevorzugt für Laktat, Sulfat, Nitrat, Thiosulfat, Thiocyanat, Citrat, Oxalat oder Formiat steht,
- R³⁰: außerdem besonders bevorzugt für Acetat, Monohydrogenphosphat oder Dihydrogenphosphat steht und
- R³⁰: ganz besonders bevorzugt für Sulfat, Thiocyanat, Dihydrogenphosphat oder Monohydrogenphosphat steht.

Die Ammonium- und Phosphoniumsalze der Formel (II') können in einem breiten Konzentrationsbereich zur Steigerung der Wirkung von Pflanzenschutzmitteln enthaltend Ketoenole eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern zusätzlich ein Penetrationsförderer zugegeben. Es ist als völlig überraschend zu bezeichnen, dass selbst in diesen Fällen eine noch weiter gehende Wirkungssteigerung zu beobachten ist. Gegenstand der vorliegenden Erfindung ist also ebenfalls die Verwendung einer Kombination von Penetrationsförderer und Ammonium- und/oder Phosphoniumsalzen zur Wirkungssteigerung von Pflanzenschutzmitteln, die herbizid wirksame 3-(2-Alkoxy-phenyl)-substituierte Tetramate als Wirkstoff enthalten. Gegenstand der Erfindung sind ebenfalls Mittel, die insektizid und/oder akarizid und/oder herbizid wirksame 3-(2-Alkoxy-phenyl)-substituierte Tetramate, Penetrationsförderer und Ammonium- und/oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von unerwünschtem Pflanzenwuchs.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässerigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-allcoxylate in Betracht. Erfindungsgemäße Penetrationsförderer sind Alkanol-alkoxylate der Formel

R-O-(-AO)ᵥ-R' (III')

in welcher
- R: für geradkettiges oder verzweigtes Alkyl mit 4 bis 20 Kohlenstoffatomen steht,
- R': für Wasserstoff, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- v: für Zahlen von 2 bis 30 steht.

Dabei werden solche Alkanol-Alkoxylate, bei denen R' Wasserstoff ist, als "offene" Alkanol-Alkoxylate bezeichnet. Eine bevorzugte Gruppe von Penetrationsförderern sind Alkanolalkoxylate der Formel

R-O-(-EO-)ₙ-R' (III'-a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (III'-b)

in welcher
- R: die oben angegebene Bedeutung hat,
- R': die oben angegebene Bedeutung hat,
- EO: für -CH₂-CH₂-O- steht,
- PO: für 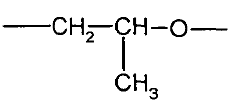 steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (III'-c)

in welcher
- R: die oben angegebene Bedeutung hat,
- R': die oben angegebene Bedeutung hat,
- EO: für -CH₂-CH₂-O- steht,
- PO: für 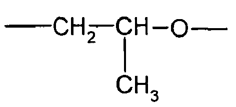 steht,
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (III'-d)

in welcher
- R und R': die oben angegebenen Bedeutungen haben,
- EO: für CH₂-CH₂-O- steht,
- BO: für 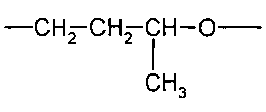 steht,
- p: für Zahlen von 1 bis 10 steht und
- q: für Zahlen von 1 bis 10 steht.

Eine weitere bevorzugte Gruppe von Penetrationsförderem sind Alkanol-alkoxylate der Formel

R-O-(-BO-)ᵣ (-EO-)ₛ-R' (III'-e)

in welcher
- R und R': die oben angegebenen Bedeutungen haben,
- BO: für 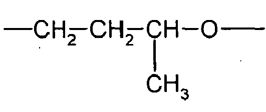 steht,
- EO: für CH₂-CH₂-O- steht,
- r: für Zahlen von 1 bis 10 steht und
- s: für Zahlen von 1 bis 10 steht.

Eine weitere bevorzugte Gruppe von Penetrationsförderern sind Alkanol-Alkoxylate der Formel

CH₃-(CH₂)ₜ-CH₂-O-(-CH₂-CH₂-O-)ᵤ-R' (III'-f)

in welcher
- R': die oben angegebene Bedeutung hat,
- t: für Zahlen von 8 bis 13 steht
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (III-c) sei 2-Ethyl-hexyl-alkoxylat der Formel 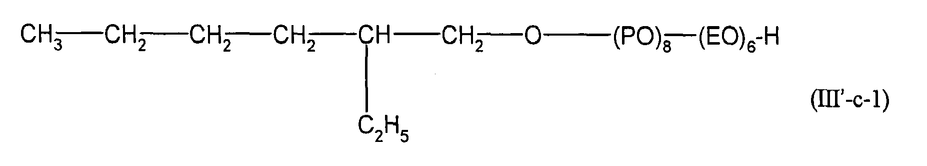 in welcher
- EO: für -CH₂-CH₂-O- steht,
- PO: für 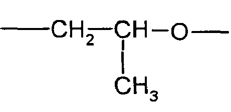 steht und
die Zahlen 8 und 6 Durchschnittswerte darstellen, genannt.

Als Beispiel für ein Alkanol-Alkoxylat der Formel (III-d) sei die Formel

CH₃-(CH₂)₁₀-O-(-EO-)₆-(-BO-)₂-CH₃ (III'-d-1)

in welcher
- EO: für CH₂-CH₂-O- steht,
- BO: für 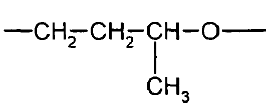 steht und
die Zahlen 10, 6 und 2 Durchschnittswerte darstellen, genannt.

Besonders bevorzugte Alkanol-Alkoxylate der Formel (III'-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (III'-f-1)

CH₃-(CH₂)ₜ-CH₂-O-(-CH₂-CH₂-O-)ᵤ-H (III'-f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98-35 553, WO 00-35 278 und EP-A 0 681 865).

Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäß hervorgehobene Kombinationen von Wirkstoff, Salz und Penetrations-förderer sind in folgender Tabelle aufgeführt. "gemäß Test" bedeutet dabei, dass jede Verbindung geeignet ist, die in dem Test für die Kutikelpenetration (Baur et al., 1997, Pesticide Science 51, 131-152) als Penetrationsförderer wirkt.

| # | Wirkstoff | Salz | Penetrationsförderer |
|---|---|---|---|
| 1 | A.1 | Ammoniumsulfat | gemäß Test |
| 2 | A.1 | Ammoniumlaktat | gemäß Test |
| 3 | A.1 | Ammoniumnitrat | gemäß Test |
| 4 | A.1 | Ammoniumthiosulfat | gemäß Test |
| 5 | A.1 | Ammoniumthiocyanat | gemäß Test |
| 6 | A.1 | Ammoniumcitrat | gemäß Test |
| 7 | A.1 | Ammoniumoxalat | gemäß Test |
| 8 | A.1 | Ammoniumformiat | gemäß Test |
| 9 | A.1 | Ammoniumhydrogenphosphat | gemäß Test |
| 10 | A.1 | Ammoniumdihydrogenphosphat | gemäß Test |
| 11 | A.1 | Ammoniumcarbonat | gemäß Test |
| 12 | A.1 | Ammoniumbenzoat | gemäß Test |
| 13 | A.1 | Amrraoniumsulfit | gemäß Test |
| 14 | A.1 | Ammoniumbenzoat | gemäß Test |
| 15 | A.1 | Ammoniumhydrogenoxalat | gemäß Test |
| 16 | A.2 | Ammoniumsulfat | gemäß Test |
| 17 | A.2 | Ammoniumlaktat | gemäß Test |
| 18 | A.2 | Ammoniumnitrat | gemäß Test |
| 19 | A.2 | Ammoniumthiosulfat | gemäß Test |
| 20 | A.2 | Ammoniumthiocyanat | gemäß Test |
| 21 | A.2 | Ammoniumcitrat | gemäß Test |
| 22 | A.2 | Ammoniumoxalat | gemäß Test |
| 23 | A.2 | Ammoniumformiat | gemäß Test |
| 24 | A.2 | Ammoniumhydrogenphosphat | gemäß Test |
| 25 | A.2 | Ammoniumdihydrogenphosphat | gemäß Test |
| 26 | A.2 | Ammoniumcarbonat | gemäß Test |
| 27 | A.2 | Ammoniumbenzoat | gemäß Test |
| 28 | A.2 | Ammoniumsulfit | gemäß Test |
| 29 | A.2 | Amnaoniumbenzoat | gemäß Test |
| 30 | A.2 | Ammoniumhydrogenoxalat | gemäß Test |
| 31 | A.3 | Ammoniumsulfat | gemäß Test |
| 32 | A.3 | Ammoniumlaktat | gemäß Test |
| 33 | A.3 | Ammoniumnitrat | gemäß Test |
| 34 | A.3 | Ammoniumthiosulfat | gemäß Test |
| 35 | A.3 | Ammoniumthiocyanat | gemäß Test |
| 36 | A.3 | Ammoniumcitrat | gemäß Test |
| 37 | A.3 | Ammoniumoxalat | gemäß Test |
| 38 | A.3 | Ammoniumformiat | gemäß Test |
| 39 | A.3 | Ammoniumhydrogenphosphat | gemäß Test |
| 40 | A.3 | Ammoniumdihydrogenphosphat | gemäß Test |
| 41 | A.3 | Ammoniumcarbonat | gemäß Test |
| 42 | A.3 | Ammoniumbenzoat | gemäß Test |
| 43 | A.3 | Ammoniumsulfit | gemäß Test |
| 44 | A.3 | Ammoniumbenzoat | gemäß Test |
| 45 | A.3 | Ammmniumhydrogenoxatat | gemäß Test |
| 46 | A.4 | Ammoniumsulfat | gemäß Test |
| 47 | A.4 | Ammoniumlaktat | gemäß Test |
| 48 | A.4 | Amrmtniumnitrat | gemäß Test |
| 49 | A.4 | Ammoniumthiosulfat | gemäß Test |
| 50 | A.4 | Ammoniumthiocyanat | gemäß Test |
| 51 | A.4 | Ammoniumcitrat | gemäß Test |
| 52 | A.4 | Ammoniumoxalat | gemäß Test |
| 53 | A.4 | Ammoniumformiat | gemäß Test |
| 54 | A.4 | Ammoniumhydrogenphosphat | gemäß Test |
| 55 | A.4 | Ammoniumdihydrogenphosphat | gemäß Test |
| 56 | A.4 | Ammoniumcarbonat | gemäß Test |
| 57 | A.4 | Ammoniumbenzoat | gemäß Test |
| 58 | A.4 | Ammoniumsulfit | gemäß Test |
| 59 | A.4 | Ammoniumbenzoat | gemäß Test |
| 60 | A.4 | Ammoniumhydrogenoxalat | gemäß Test |
| 61 | A.5 | Ammoniumsulfat | gemäß Test |
| 62 | A.5 | Ammoniumtaktat | gemäß Test |
| 63 | A.5 | Ammoniumnitrat | gemäß Test |
| 64 | A.5 | Ammoniumthiosulfat | gemäß Test |
| 65 | A.5 | Ammoniumthiocyanat | gemäß Test |
| 66 | A.5 | Ammoniumcitrat | gemäß Test |
| 67 | A.5 | Ammoniumoxalat | gemäß Test |
| 68 | A.5 | Ammoniumformiat | gemäß Test |
| 69 | A.5 | Ammoniumhydrogenphosphat | gemäß Test |
| 70 | A.5 | Ammoniumdihydrogenphosphat | gemäß Test |
| 71 | A.5 | Ammoniumcarbonat | gemäß Test |
| 72 | A.5 | Ammoniumbenzoat | gemäß Test |
| 73 | A.5 | Ammoniumsulfit | gemäß Test |
| 74 | A.5 | Ammoniumbenzoat | gemäß Test |
| 75 | A.5 | Ammoniumhydrogenoxalat | gemäß Test |
| 76 | A.6 | Ammoniumsulfat | gemäß Test |
| 77 | A.6 | Ammoniumlaktat | gemäß Test |
| 78 | A.6 | Ammoniumnitrat | gemäß Test |
| 79 | A.6 | Ammoniumthiosulfat | gemäß Test |
| 80 | A.6 | Ammoniumthiocyanat | gemäß Test |
| 81 | A.6 | Ammoniumcitrat | gemäß Test |
| 82 | A.6 | Ammoniumoxalat | gemäß Test |
| 83 | A.6 | Ammoniumformiat | gemäß Test |
| 84 | A.6 | Ammoniumhydrogenphosphat | gemäß Test |
| 85 | A.6 | Ammoniumdihydrogenphosphat | gemäß Test |
| 86 | A.6 | Ammoniumcarbonat | gemäß Test |
| 87 | A.6 | Ammoniumbenzoat | gemäß Test |
| 88 | A.6 | Ammoniumsulfit | gemäß Test |
| 89 | A.6 | Ammoniumbenzoat | gemäß Test |
| 90 | A.6 | Ammoniumhydrogenoxalat | gemäß Test |
| 91 | A.7 | Ammoniumsulfat | gemäß Test |
| 92 | A.7 | Ammoniumlaktat | gemäß Test |
| 93 | A.7 | Ammoniumnitrat | gemäß Test |
| 94 | A.7 | Ammoniumthiosulfat | gemäß Test |
| 95 | A.7 | Ammoniumthiocyanat | gemäß Test |
| 96 | A.7 | Ammoniumcitrat | gemäß Test |
| 97 | A.7 | Ammoniumoxalat | gemäß Test |
| 98 | A.7 | Ammoniumformiat | gemäß Test |
| 99 | A.7 | Ammoniumhydrogenphosphat | gemäß Test |
| 100 | A.7 | Ammoniumdihydrogenphosphat | gemäß Test |
| 101 | A.7 | Ammoniumcarbonat | gemäß Test |
| 102 | A.7 | Ammoniumbenzoat | gemäß Test |
| 103 | A.7 | Ammoniumsulfit | gemäß Test |
| 104 | A.7 | Ammoniumbenzoat | gemäß Test |
| 105 | A.7 | Ammoniumhydrogenoxalat | gemäß Test |
| 106 | A.8 | Ammoniumsulfat | gemäß Test |
| 107 | A.8 | Ammoniumlaktat | gemäß Test |
| 108 | A.8 | Ammoniumnitrat | gemäß Test |
| 109 | A.8 | Ammoniumthiosulfat | gemäß Test |
| 110 | A.8 | Ammoniumthiocyanat | gemäß Test |
| 111 | A.8 | Ammoniumcitrat | gemäß Test |
| 112 | A.8 | Ammoniumoxalat | gemäß Test |
| 113 | A.8 | Ammoniumformiat | gemäß Test |
| 114 | A.8 | Ammoniumhydrogenphosphat | gemäß Test |
| 115 | A.8 | Ammoniumdihydrogenphosphat | gemäß Test |
| 116 | A.8 | Ammoniumcarbonat | gemäß Test |
| 117 | A.8 | Ammoniumbenzoat | gemäß Test |
| 118 | A.8 | Ammoniumsulfit | gemäß Test |
| 119 | A.8 | Ammoniumbenzoat | gemäß Test |
| 120 | A.8 | Ammoniumhydrogenoxalat | gemäß Test |
| 121 | A.9 | Ammoniumsulfat | gemäß Test |
| 122 | A.9 | Ammoniumlaktat | gemäß Test |
| 123 | A.9 | Ammoniumnitrat | gemäß Test |
| 124 | A.9 | Ammoniumthiosulfat | gemäß Test |
| 125 | A.9 | Ammoniumthiocyanat | gemäß Test |
| 126 | A.9 | Ammoniumcitrat | gemäß Test |
| 127 | A.9 | Ammoniumoxalat | gemäß Test |
| 128 | A.9 | Ammoniumformiat | gemäß Test |
| 129 | A.9 | Ammoniumhydrogenphosphat | gemäß Test |
| 130 | A.9 | Ammoniumdihydrogenphosphat | gemäß Test |
| 131 | A.9 | Ammoniumcarbonat | gemäß Test |
| 132 | A.9 | Ammoniumbenzoat | gemäß Test |
| 133 | A.9 | Ammoniumsulfit | gemäß Test |
| 134 | A.9 | Ammoniumbenzoat | gemäß Test |
| 135 | A.9 | Ammoniumhydrogenoxalat | gemäß Test |
| 136 | A.10 | Ammoniumsulfat | gemäß Test |
| 137 | A.10 | Ammoniumlaktat | gemäß Test |
| 138 | A.10 | Ammoniumnitrat | gemäß Test |
| 139 | A.10 | Ammoniumthiosulfat | gemäß Test |
| 140 | A.10 | Ammoniumthiocyanat | gemäß Test |
| 141 | A.10 | Ammoniumcitrat | gemäß Test |
| 142 | A.10 | Ammoniumoxalat | gemäß Test |
| 143 | A.10 | Ammoniumformiat | gemäß Test |
| 144 | A.10 | Ammoniumhydrogenphosphat | gemäß Test |
| 145 | A.10 | Ammoniumdihydrogenphosphat | gemäß Test |
| 146 | A.10 | Ammoniumcarbonat | gemäß Test |
| 147 | A.10 | Ammoniumbenzoat | gemäß Test |
| 148 | A.10 | Ammoniumsulfit | gemäß Test |
| 149 | A.10 | Ammoniumbenzoat | gemäß Test |
| 150 | A.10 | Ammoniumhydrogenoxalat | gemäß Test |
| 151 | A.11 | Ammoniumsulfat | gemäß Test |
| 152 | A.11 | Ammoniumlaktat | gemäß Test |
| 153 | A.11 | Ammoniumnitrat | gemäß Test |
| 154 | A.11 | Ammoniumthiosulfat | gemäß Test |
| 155 | A.11 | Ammoniumthiocyanat | gemäß Test |
| 156 | A.11 | Ammoniumcitrat | gemäß Test |
| 157 | A.11 | Ammoniumoxalat | gemäß Test |
| 158 | A.11 | Ammoniumformiat | gemäß Test |
| 159 | A.11 | Ammoniumhydrogenphosphat | gemäß Test |
| 160 | A.11 | Ammoniumdihydrogenphosphat | gemäß Test |
| 161 | A.11 | Ammoniumcarbonat | gemäß Test |
| 162 | A.11 | Ammoniumbenzoat | gemäß Test |
| 163 | A.11 | Ammoniumsulfit | gemäß Test |
| 164 | A.11 | Ammoniumbenzoat | gemäß Test |
| 165 | A.11 | Ammoniumhydrogenoxalat | gemäß Test |
| 166 | A.12 | Ammoniumsulfat | gemäß Test |
| 167 | A.12 | Ammoniumlaktat | gemäß Test |
| 168 | A.12 | Ammoniumnitrat | gemäß Test |
| 169 | A.12 | Ammoniumthiosulfat | gemäß Test |
| 170 | A.12 | Ammoniumthiocyanat | gemäß Test |
| 171 | A.12 | Ammoniumcitrat | gemäß Test |
| 172 | A.12 | Ammoniumoxalat | gemäß Test |
| 173 | A.12 | Ammoniumformiat | gemäß Test |
| 174 | A.12 | Ammoniumhydrogenphosphat | gemäß Test |
| 175 | A.12 | Ammoniumdihydrogenphosphat | gemäß Test |
| 176 | A.12 | Ammoniumcarbonat | gemäß Test |
| 177 | A.12 | Ammoniumbenzoat | gemäß Test |
| 178 | A.12 | Ammoniumsulfit | gemäß Test |
| 179 | A.12 | Ammoniumbenzoat | gemäß Test |
| 180 | A.12 | Ammoniumhydrogenoxalat | gemäß Test |
| 181 | A.13 | Ammoniumsulfat | gemäß Test |
| 182 | A.13 | Ammoniumlaktat | gemäß Test |
| 183 | A.13 | Ammoniumnitrat | gemäß Test |
| 184 | A.13 | Ammoniumthiosulfat | gemäß Test |
| 185 | A.13 | Ammoniumthiocyanat | gemäß Test |
| 186 | A.13 | Ammoniumcitrat | gemäß Test |
| 187 | A.13 | Ammoniumoxalat | gemäß Test |
| 188 | A.13 | Ammoniumformiat | gemäß Test |
| 189 | A.13 | Ammoniumhydrogenphosphat | gemäß Test |
| 190 | A.13 | Ammoniumdihydrogenphosphat | gemäß Test |
| 191 | A.13 | Ammoniumcarbonat | gemäß Test |
| 192 | A.13 | Ammoniumbenzoat | gemäß Test |
| 193 | A.13 | Ammoniumsulfit | gemäß Test |
| 194 | A.13 | Ammoniumbenzoat | gemäß Test |
| 195 | A.13 | Ammoniumhydrogenoxalat | gemäß Test |
| 196 | A.14 | Ammoniumsulfat | gemäß Test |
| 197 | A.14 | Ammoniumlaktat | gemäß Test |
| 198 | A.14 | Ammoniumnitrat | gemäß Test |
| 199 | A.14 | Ammoniumthiosulfat | gemäß Test |
| 200 | A.14 | Ammoniumthiocyanat | gemäß Test |
| 201 | A.14 | Ammoniumcitrat | gemäß Test |
| 202 | A.14 | Ammoniumoxalat | gemäß Test |
| 203 | A.14 | Ammoniumformiat | gemäß Test |
| 204 | A.14 | Ammoniumhydrogenphosphat | gemäß Test |
| 205 | A.14 | Ammoniumdihydrogenphosphat | gemäß Test |
| 206 | A.14 | Ammoniumcarbonat | gemäß Test |
| 207 | A.14 | Ammoniumbenzoat | gemäß Test |
| 208 | A.14 | Ammoniumsulfit | gemäß Test |
| 209 | A.14 | Ammoniumbenzoat | gemäß Test |
| 210 | A.14 | Ammoniumhydrogenoxalat | gemäß Test |
| 211 | A.15 | Ammoniumsulfat | gemäß Test |
| 212 | A.15 | Ammoniumlaktat | gemäß Test |
| 213 | A.15 | Ammoniumnitrat | gemäß Test |
| 214 | A.15 | Ammoniumthiosulfat | gemäß Test |
| 215 | A.15 | Ammoniumthiocyanat | gemäß Test |
| 216 | A.15 | Ammoniumcitrat | gemäß Test |
| 217 | A.15 | Ammoniumoxalat | gemäß Test |
| 218 | A.15 | Ammoniumformiat | gemäß Test |
| 219 | A.15 | Ammoniumhydrogenphosphat | gemäß Test |
| 220 | A.15 | Ammoniumdihydrogenphosphat | gemäß Test |
| 221 | A.15 | Ammoniumcarbonat | gemäß Test |
| 222 | A.15 | Ammoniumbenzoat | gemäß Test |
| 223 | A.15 | Ammoniumsulfit | gemäß Test |
| 224 | A.15 | Ammoniumbenzoat. | gemäß Test |
| 225 | A.15 | Ammoniumhydrogenoxalat | gemäß Test |
| 226 | A.16 | Ammoniumsulfat | gemäß Test |
| 227 | A.16 | Ammoniumlaktat | gemäß Test |
| 228 | A.16 | Ammoniumnitrat | gemäß Test |
| 229 | A.16 | Ammoniumthiosulfat | gemäß Test |
| 230 | A.16 | Ammoniumthiocyanat | gemäß Test |
| 231 | A.16 | Ammoniumcitrat | gemäß Test |
| 232 | A.16 | Ammoniumoxalat | gemäß Test |
| 233 | A.16 | Ammoniumformiat | gemäß Test |
| 234 | A.16 | Ammoniumhydrogenphosphat | gemäß Test |
| 235 | A.16 | Ammoniumdihydrogenphosphat | gemäß Test |
| 236 | A.16 | Ammoniumcarbonat | gemäß Test |
| 237 | A.16 | Ammoniumbenzoat | gemäß Test |
| 238 | A.16 | Ammoniumsulfit | gemäß Test |
| 239 | A.16 | Ammoniumbenzoat | gemäß Test |
| 240 | A.16 | Ammoniumhydrogenoxalat | gemäß Test |
| 241 | A.17 | Ammoniumsulfat | gemäß Test |
| 242 | A.17 | Ammoniumlaktat | gemäß Test |
| 243 | A.17 | Ammoniumnitrat | gemäß Test |
| 244 | A.17 | Ammoniumthiosulfat | gemäß Test |
| 245 | A.17 | Ammoniumthiocyanat | gemäß Test |
| 246 | A.17 | Ammoniumcitrat | gemäß Test |
| 247 | A.17 | Ammoniumoxalat | gemäß Test |
| 248 | A.17 | Ammoniumformiat | gemäß Test |
| 249 | A.17 | Ammoniumhydrogenphosphat | gemäß Test |
| 250 | A.17 | Ammoniumdihydrogenphosphat | gemäß Test |
| 251 | A.17 | Ammoniumcarbonat | gemäß Test |
| 252 | A.17 | Ammoniumbenzoat | gemäß Test |
| 253 | A.17 | Ammoniumsulfit | gemäß Test |
| 254 | A.17 | Ammoniumbenzoat | gemäß Test |
| 255 | A.17 | Ammoniumhydrögenoxalat | gemäß Test |
| 256 | A.18 | Ammoniumsulfat | gemäß Test |
| 257 | A.18 | Ammoniumtaktat | gemäß Test |
| 258 | A.18 | Ammoniumnitrat | gemäß Test |
| 259 | A.18 | Ammoniumthiosulfat | gemäß Test |
| 260 | A.18 | Ammoniumthiocyanat | gemäß Test |
| 261 | A.18 | Ammoniumcitrat | gemäß Test |
| 262 | A.18 | Ammoniumoxalat | gemäß Test |
| 263 | A.18 | Ammoniumformiat | gemäß Test |
| 264 | A.18 | Ammoniumhydrogenphosphat | gemäß Test |
| 265 | A.18 | Ammoniumdihydrogenphosphat | gemäß Test |
| 266 | A.18 | Ammoniumcarbonat | gemäß Test |
| 267 | A.18 | Ammoniumbenzoat | gemäß Test |
| 268 | A.18 | Ammoniumsulfit | gemäß Test |
| 269 | A.18 | Ammoniumbenzoat | gemäß Test |
| 270 | A.18 | Ammoniumhydrogenoxalat | gemäß Test |
| 271 | A.19 | Ammoniumsulfat | gemäß Test |
| 272 | A.19 | Ammoniumlaktat | gemäß Test |
| 273 | A.19 | Ammoniumnitrat | gemäß Test |
| 274 | A.19 | Ammoniumthiosulfat | gemäß Test |
| 275 | A.19 | Ammoniumthiocyanat | gemäß Test |
| 276 | A.19 | Ammoniumcitrat | gemäß Test |
| 277 | A.19 | Ammoniumoxalat | gemäß Test |
| 278 | A.19 | Ammoniumformiat | gemäß Test |
| 279 | A.19 | Ammoniumhydrogenphosphat | gemäß Test |
| 280 | A.19 | Ammoniumdihydrogenphosphat | gemäß Test |
| 281 | A.19 | Ammoniumcarbonat | gemäß Test |
| 282 | A.19 | Ammoniumbenzoat | gemäß Test |
| 283 | A.19 | Ammoniumsulfit | gemäß Test |
| 284 | A.19 | Ammoniumbenzoat | gemäß Test |
| 285 | A.19 | Ammoniumhydrogenoxalat | gemäß Test |
| 286 | A.20 | Ammoniumsulfat | gemäß Test |
| 287 | A.20 | Ammoniumlaktat | gemäß Test |
| 288 | A.20 | Ammoniumnitrat | gemäß Test |
| 289 | A.20 | Ammoniumthiosulfat | gemäß Test |
| 290 | A.20 | Ammoniumthiocyanat | gemäß Test |
| 291 | A.20 | Ammoniumcitrat | gemäß Test |
| 292 | A.20 | Ammoniumoxalat | gemäß Test |
| 293 | A.20 | Ammoniumformiat | gemäß Test |
| 294 | A.20 | Ammoniumhydrogenphosphat | gemäß Test |
| 295 | A.20 | Ammoniumdihydrogenphosphat | gemäß Test |
| 296 | A.20 | Ammoniumcarbonat | gemäß Test |
| 297 | A.20 | Ammoniumbenzoat | gemäß Test |
| 298 | A.20 | Ammoniumsulfit | gemäß Test |
| 299 | A.20 | Ammoniumbenzoat | gemäß Test |
| 300 | A.20 | Ammoniumhydrogenoxalat | gemäß Test |
| 301 | A.21 | Ammoniumsulfat | gemäß Test |
| 302 | A.21 | Ammoniumlaktat | gemäß Test |
| 303 | A.21 | Ammoniumnitrat | gemäß Test |
| 304 | A.21 | Ammoniumthiosulfat | gemäß Test |
| 305 | A.21 | Ammoniumthiocyanat | gemäß Test |
| 306 | A.21 | Ammoniumcitrat | gemäß Test |
| 307 | A.21 | Ammoniumoxalat | gemäß Test |
| 308 | A.21 | Ammoniumformiat | gemäß Test |
| 309 | A.21 | Ammoniumhydrogenphosphat | gemäß Test |
| 310 | A.21 | Ammoniumdihydrogenphosphat | gemäß Test |
| 311 | A.21 | Ammoniumcarbonat | gemäß Test |
| 312 | A.21 | Ammoniumbenzoat | gemäß Test |
| 313 | A.21 | Ammoniumsulfit | gemäß Test |
| 314 | A.21 | Ammoniumbenzoat | gemäß Test |
| 315 | A.21 | Ammoniumhydrogenoxalat | gemäß Test |

Verwendet man beispielsweise gemäß Verfahren (A) N-(2-Methyl-4-chlor-6-meihoxy-phenylacetyl)-N-cyclopropy-glycin-ethylester als Ausgangsstoff, so kann der Verlauf des erfindungsgemäßen Verfahrens durch folgendes Reaktionsschema wiedergegeben werden:

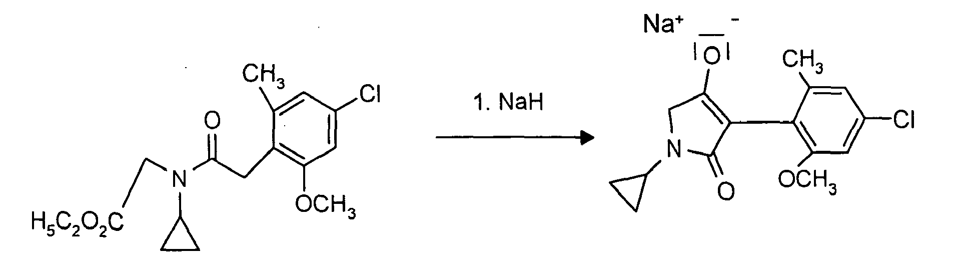

Verwendet man beispielsweise gemäß Verfahren (B) 3-(2-Ehyl-4-chlor-6-methoxy-phenyl]-5,5-dimethyl-pyrrolidin-2,4-dion und NaOH als Komponenten, so kann der Verlauf des erfindungsgemäßen Verfahrens durch folgendes Reaktionsschema wiedergegeben werden:

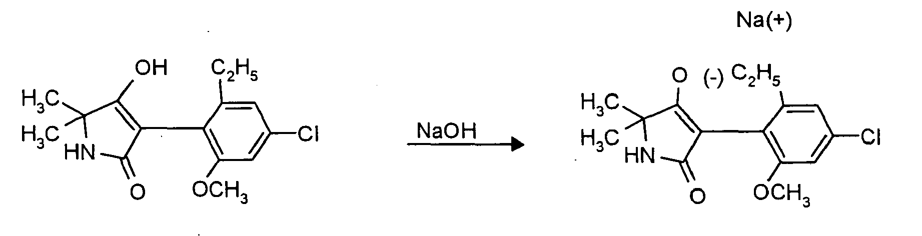

Die beim erfindungsgemäßen Verfahren (a) als Ausgangsstoffe benötigten Verbindungen der Formel (II) 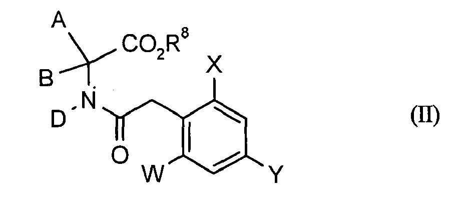 in welcher
- A, B, D, W, X, Y und R⁸: die oben angegebenen Bedeutungen haben,
sind teilweise bekannt aus WO 04/080962 oder lassen sich nach den dort beschriebenen Verfahren herstellen.

Die zur Durchführung des erfindungsgemäßen Verfahren (B) außerdem als Ausgangsstoffe benötigten Metallhydroxide, Metallalkoxide oder Metallhydride der Formeln (III) oder (IV) sind allgemein bekannte Verbindungen der Anorganischen Chemie.

Die Verbindungen der Formeln (I') und (II) sind darüber hinaus aus den eingangs zitierten Patentanmeldungen bekannt und/oder lassen sich nach den dort angegebenen Methoden herstellen.

Das Verfahren (A) ist dadurch gekennzeichnet, dass man Verbindungen der Formel (II), in welcher A, B, D, W, X, Y und R² die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, IsoPropanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (A) wird im Allgemeinen unter Normaldruck durchgeführt.

Bei der Durchführung des erfindungsgemäßen Verfahrens (A) setzt man die Reaktionskomponenten der Formel (II) und die deprotonierenden Basen im Allgemeinen in etwa doppeläquimolaren Mengen ein. Es ist jedoch auch möglich, die eine oder andere Komponente in einem größeren Überschuss (bis zu 3 Mol) zu verwenden.

Das Verfahren (B) ist dadurch gekennzeichnet, dass man Verbindungen der Formel (I') mit Metallhydroxiden bzw. Metallalkoxiden der Formel (III) oder Metallhydriden der Formel (IV), gegebenenfalls in Gegenwart eines Verdünnungsmittels, umsetzt.

Als Verdünnungsmittel können bei dem erfindungsgemäßen Verfahren (B) vorzugsweise Ether wie Tetrahydrofuran, Dioxan, Diethylether oder aber Alkohole wie Methanol, Ethanol, Isopropanol, aber auch Wasser eingesetzt werden.

Das erfindungsgemäße Verfahren (B) wird im Allgemeinen unter Normaldruck durchgeführt.

Die Reaktionstemperaturen liegen im Allgemeinen zwischen -20°C und 100°C, vorzugsweise zwischen 0°C und 50°C.

Die erfindungsgemäßen Wirkstoffe/Wirkstoffkombinationen eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp.

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Omithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp.

Aus der Ordnung der Chilopoda z.B. Geophilus spp., Scutigera spp.

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp.

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa.

Aus der Klasse der Gastropoda z.B. Arion spp., Biomphalaria spp., Bulinus spp., Deroceras spp., Galba spp., Lymnaea spp., Oncomelania spp., Succinea spp.

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp.

Aus der Ordnung der Isopoda z.B. Armadillidium vulgare, Oniscus asellus, Porcellio scaber.

Aus der Ordnung der Isoptera z.B. Reticulitermes spp.

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp.

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Rhipiphorothrips cruentatus, Scirtothrips spp., Taeniothrips cardamoni, Thrips spp.

Aus der Ordnung der Thysanura z.B. Lepisma saccharina.

Zu den pflanzenparasitären Nematoden gehören z.B. Aphelenchoides spp., Bursaphelenchus spp., Ditylenchus dipsaci, Globodera spp., Heterodera spp., Longidorus spp., Meloidogyne spp., Pratylenchus spp., Radopholus similis, Trichodorus spp., Tylenchulus semipenetrans, Xiphinema spp.

Die erfindungsgemäßen Verbindungen/Wirkstoffkombinationen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen/Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Samen, weiterhin durch ein- oder mehrschichtiges Umhüllen.

Die Wirkstoffe/Wirkstoffkombinationen können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe/Wirkstoffkombinationen mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gumtniarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gew.-% Wirkstoff, vorzugsweise zwischen 0,5 und 90 %.

Der erfindungsgemäße Wirkstoff/Wirkstoffkombinationen kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe/Wirkstoftlcombinationen können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muss.

Die erfindungsgemäßen Wirkstoffe/Wirkstoffkombinationen können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepassten üblichen Weise.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausfiihrungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders. hervorgehoben sei die Pflanzenbehandlung mit den im vorliegenden Text speziell aufgeführten Verbindungen bzw. Mischungen.

Die erfindungsgemäßen Wirkstoffe/Wirkstoffkombinationen wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp.

Aus der Unterklasse der Acari (Acarina) und den Ordnungen der Meta- sowie Mesostigmata z.B. Argas spp., Omithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ormthocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe/Wirkstoffkombinationen der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe/Wirkstoffkombinationen geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen/Wirkstoffkombinationen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychas brunneus, Sinoxylon spec. Dincderus minutus;

Hautflügler wie Sirex juvencus, Urocerus gigas, Urocerus gigas taignus, Urocerus augur;

Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im Allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausfuhrungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl-oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinyhnethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Itnprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid, Triflumuron, Chlothianidin, Spinosad, Tefluthrin,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen; insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis(trialkylzinn)-sulfiden, Tri-*n-*butylzinnlaurat, Tri-*n*-butylzinnchlorid, Kupfer(I)-oxid, Triethylzinnchlorid, Tri-*n*-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithio-carbamat, Kupferthiocyanat, Kupfemaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:

Algizide wie
2-*tert.*-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;

Fungizide wie
Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie
Azaconazole, Cyproconazole, Epoxyconazole, Hexaconazole, Metconazole, Propiconazole und Tebuconazole;

Molluskizide wie
Fentinacetat, Metaldehyd, Methiocarb, Niclosamid, Thiodicarb und Trimethacarb, Fe-chelate;
oder herkömmliche Antifouling-Wirkstoffe wie
4,5-Dichlor-2-octyl-4-isothiazolin-3-on, Düodmethylparatrylsulfon, 2-(N,N-Dimethylthiocarbamoyl-thio)-5-nitrothiazyl, Kalium-, Kupfer-, Natrium- und Zinksalze von 2-Pyridinthiol-1-oxid, Pyridintriphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Siyrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Omithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp.

Aus der Ordnung der Chilopoda z.B. Geophilus spp.

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coloptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga camaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe/Wirkstoffkombinationen können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe/Wirkstoffkombinationen können z.B. bei den folgenden Pflanzen verwendet werden:

Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.

Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe/Wirkstoffkombinationen ist jedoch keineswegs auf diese Gattungen beschränkt, sondern erstreckt sich in gleicher Weise auch auf andere Pflanzen.

Die erfindungsgemäßen Wirkstoffe/Wirkstoffkombinationen eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I)/Wirkstofflcombinationen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe/Wirkstoffkombinationen können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe/Wirkstoffkombinationen können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe/Wirkstoffkombinationen können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe/Wirkstoffkombinationen können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe/Wirkstoffkombinationen können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im Allgemeinen entfallen auf 1 Gewichtsteil Wirkstoff der Formel (I) 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im Allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im Allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäßen Wirkstofflcombinationen können vor und nach dem Auflaufen der Pflanzen appliziert werden, also im Vorauflauf und Nachauflauf-Verfahren.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Die Herstellung und Verwendung der erfindungsgemäßen Wirkstoffe/Wirkstoffkombinationen geht aus den nachfolgenden Beispielen hervor.

### Herstellungsbeispiele

### Beispiel A.1

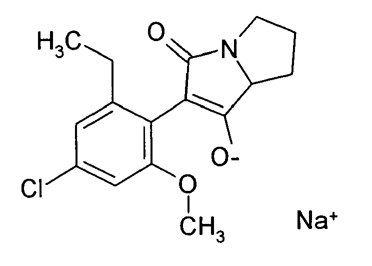

0,42ml einer 30%-igen Natriummethylatlösung vorgelegt und 0,5g der Verbindung gemäß Bsp. 1-1-a-16 (WO 04-080962) in 5 ml Methanol zugeben, 1h bei Raumtemperatur rühren, das Lösungsmittel abrotieren und trocknen.

Ausbeute: 0,52g (97% d. Theorie)

¹H-NMR (300 MHz, D₂O): δ =3.93 (m, 1H, C(5)H-N),
6.92 und 6.97 (d, je 1H, Ar-H) ppm.

In Analogie zu Beispiel A.1 und gemäß den allgemeinen Angaben zur Herstellung von Verbindungen der Formel (I) erhält man folgende Beispiele:

| | | | | | | | | | | |
|---|---|---|---|---|---|---|---|---|---|---|
| 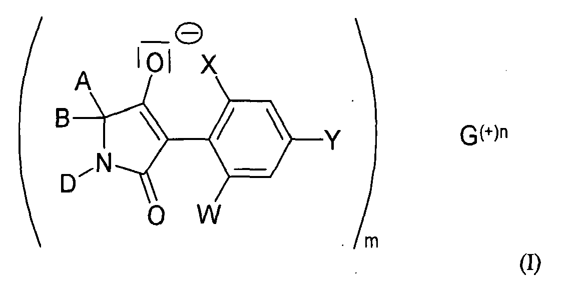 | | | | | | | | | | |

| Bsp. Nr. | W | X | Y | **D** | **A** | **B** | **G⁽⁺⁾ₙ** | **m** | **Fp.°C** | **Isomer** |
|---|---|---|---|---|---|---|---|---|---|---|
| A.2 | C₂H₅ | OCH₃ | C1 | -(CH₂)₃- | | H | Li⁺ | 1 | *3.95 (m, 1H, C(5)H-N), 6.93 und 6.99 (d, je 1H, Ar-H) | - |
| A.3 | C₂H₅ | OCH₃ | C1 | -(CH₂)₃- | | H | K⁺ | 1 | *3.96 (m, 1H, C(5)H-N), 6.93 und 6.99 (d, je 1H, Ar-H) | - |
| A.4 | C₂H₅ | OCH₃ | C1 | H | 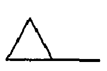 | CH₃ | Na⁺ | 1 | *1.13 (m, 1H, CH-cycPr), 6.95 und 7.00 (d, je 1H, Ar-H) | - |
| A.5 | C₂H₅ | OCH₃ | C1 | H | 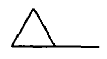 | CH₃ | K⁺ | 1 | *1.13 (m, 1H, CH-cycPr), 6.93 und 7.00 (d, je 1H, Ar-H) | - |
| A.6 | C₂H₅ | OC₂H₅ | C1 | H | -(CH₂)₂-CHOCH₃-(CH₂)₂-CH | | Na+ | 1 | **0.97 und 1.15 (je t, je 3H); 3.81 (mc, 2H), 6.62 und 6.68 (je mc, je 1H) | cis |
| A.7 | C₂H₅ | OCH₃ | Cl | H | -(CH₂)₂-CHOCH₃-(₂)₂- | | Na+ | 1 | **0.95 (t, 3H), 3.58 (s, 3H), 6.64 und 6.68 (je me, je 1H) | cis |
| A.8 | C₂H₅ | OCH₃ | Cl | H | -(CH₂)₂-O-(CH₂)₂- | | Na+ | 1 | 88 | - |
| A.9 | C₂H₅ | OCH₃ | Cl | H | CH₃ | CH₃ | Na+ | 1 | **0.97 (t, 3H), 1.05 (s, 6H), 6.65 und 6.69 (je me, je 1H) | - |
| A.10 | C₂H₅ | OC₂H₅ | Cl | H | -(CH₂)₂-CHOCH₃-(CH₂)₂- | | K⁺ | 1 | **0.98 (t, 3H), 1.15 (t, 3H), 3.02, mc, 1H), 6.61 und 6.67 (je mc, je 1H) | cis |
| A.11 | C₂H₅ | OCH₃ | Cl | H | -(CH₂)₂-CHOCH₃-(CH₂)₂- | | K⁺ | 1 | **0.94 (t, 3H), 1.15-1.43 (m, 4H), 1.62 (m, 2H, 1.88 (mc, 2H), 6.65 und 6.67 (je mc, je 1H) | cis |
| A.12 | C₂H₅ | OCH₃ | Cl | H | -(CH)₂-O-(CH₂)₂- | | K⁺ | 1 | **0.99, (t, 3H, CH₂-CH₃) 1.05-1.13 (m, 2H, CH₂-CH₂-O) 1.87-1.95 (zm, 2H, CH₂-CH₂-O) 3.59 (s, 3H, OCH₃) 6.64, 6.68 (2 d , 2H, Ar-H) | - |
| A.13 | C₂H₅ | OCH₃ | Cl | H | CH₃ | CH₃ | K⁺ | 1 | **1.04 und 1.05 (je s, je 3H), 6.64 und 6.68 (je mc, je 1H) | - |
| A.14 | C₂H₅ | OC₂H₅ | Cl | H | -(CH₂)₂-CHOCH₃-(CH₂)₂- | | Li⁺ | 1 | **0.97 (t, 3H), 1.16 (t, 3H), 1.66 und 1.89 (je mc, je 2H), 6:63 und 6.68 (je mc, je 1H) | cis |
| A.15 | C₂H₅ | OCH₃ | Cl | H | -(CH₂)₂-CHOCH₃-(CH₂)₂- | | Li⁺ | 1 | **0.96(t,3H),3.15 (mc, 3H), 3.56 (mc, 3H), 4.10 (mc, 1H); 6.65 und 6.69 (je mc, je 1H) | cis |
| A.16 | C₂H₅ | OCH₃ | Cl | H | -(CH₂)₂-O-(CH₂)₂- | | Li⁺ | 1 | 342 | - |
| A.17 | C₂H₅ | OCH₃ | Cl | H | CH₃ | CH₃ | Li⁺ | 1 | **0.97 (t, 3H), 1.05 (s, 6H), 2.38-2.57 (m, 2H), 6.66 und 6.69 (je mc, je 1H) | - |
| A.18 | C₂H₅ | OC₂H₅ | Cl | H | -(CH₂)₂-CHOCH₃-(CH₂)₂- | | Ca²⁺ | 2 | **0.98 und 1.16 (je t, je 3H), 3.82, mc, 2H), 5.70-5.95 (s, br, 1H); 6.67 und 6.71 (je mc, je 1H) | cis |
| A.19 | C₂H₅ | OCH₃ | Cl | H | -(CH₂)₂-CHOCH₃-(CH₂)₂- | | Ca²⁺ | 2 | **0.96 (t, 3H), 3.07 (mc, 1H), 3.56 (s, 3 H), 6.68 und 6.71 (je mc, je 1H) | cis |
| A.20 | C₂H₅ | OCH₃ | Cl | H | CH₃ | CH₃ | Ca²⁺ | 2 | **0.98 (t, 3H), 1.11 (s, 6H), 3.59 (s, 3H); 6.69 und 7.02 (je mc, je 1H) | - |
| A.21 | C₂H₅ | OCH₃ | Cl | H | -(CH₂)₂-O-(CH₂)₂- | | MgBr⁺ | 1 | ** 1.01 (t, 3 H, CH₂CH₃), 1.10-1.17 (m, 2H, -CH₂-), 1.95-2.07 (m, 2H, -CH₂-), 3.49-3.55 (m, 2H, OCH₂), 3.60 (s, 3H, OCH₃), 3.88-3.90 (m, 2H, OCH₂), 6.69, 6.74 (2d,, 2H, Ar-H) | - |
| A.22 | C₂H₅ | OCH₃ | Cl | H | -(CH₂)₂-CHOCH₃-(CH₂)₂- | | Mg²⁺ | 2 | **0.97 (t, 3H), 3.58 (s, 3H), 6.58 und 6.69 (je s, br, je 1H) | |
| A.23 | C₂H₅ | OC₂H₅ | Cl | H | -(CH₂)₂-CHOCH₃-(CH₂)₂- | | Mg ²⁺ | 2 | 269-270 | |
| A.24 | C₂H₅ | OCH₃ | Cl | H | CH₃ | CH₃ | Mg²⁺ | 2 | **3.60 (s, 3H), 6.70 und 6.72 (je s, je 1H) | |

| | | | | | | | | | | |
|---|---|---|---|---|---|---|---|---|---|---|
| * ¹H-NMR (300 MHz, D₂O): Verschiebungen δ in ppm **¹H-NMR (400 MHz, DMSO-d6): Verschiebungen δ in ppm | | | | | | | | | | |

In Analogie zu Beispiel A. 1 und gemäß den allgemeinen Angaben zur Herstellung von Verbindungen der Formel (I) erhält man folgende Beispiele:

| | | | | | | | | | | |
|---|---|---|---|---|---|---|---|---|---|---|
| 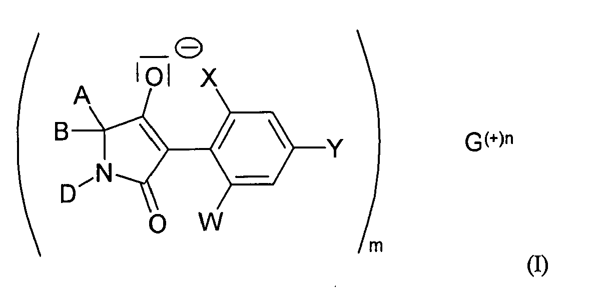 | | | | | | | | | | |

| **Bsp. Nr.** | **W** | **X** | **Y** | **D** | **A** | **B** | **G⁽⁺⁾n** | **m** | **Fp.°C** | **Isomer** |
|---|---|---|---|---|---|---|---|---|---|---|
| A.2 | C₂H₅ | OCH₃ | Cl | -(CH₂)₃- | | H | Li⁺ | 1 | *3.95 (m, 1H, C(5)H-N), 6.93 und 6.99 (d, je 1H, Ar-H) | - |
| A.3 | C₂H₅ | OCH₃ | Cl | -(CH₂)₃- | | H | K⁺ | 1 | *3.96 (m, 1H, C(5)H-N), 6.93 und 6.99 (d, je 1H, Ar-H) | - |
| A.4 | C₂H₅ | OCH₃ | Cl | H | 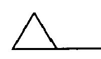 | CH₃ | Na⁺ | 1 | *1.13 (m, 1H, CH-cycPr), 6.95 und 7.00 (d, je 1H, Ar-H) | - |
| A.5 | C₂H₅ | OCH₃ | Cl | H | 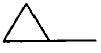 | CH₃ | K⁺ | 1 | *1.13 (m, 1H, CH-cycPr), 6.93 und 7.00 (d, je 1H, Ar-H) | - |
| A.6 | C₂H₅ | OC₂H₅ | Cl | H | -(CH₂)₂-CHOCH₃-(CH₂)₂- | | Na+ | 1 | **0.97 und 1.15 (je t, je 3H); 3.81 (mc, 2H), 6.62 und 6.68 | cis |

### Anwendungsbeispiele

### Beispiel Nr. 1

### Myzus-Test (Spritzbehandlung)

- Lösungsmittel:: 78 Gewichtsteile Aceton
1,5 Gewichtsteile Dimethylformamid
- Emulgator:: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 500 g/ha:
Bsp. Nr. A.6, A.7, A.11, A.10, A.15, A.14, A. 8

### Beispiel Nr. 2

### Tetranychus-Test, OP-resistent (Spritzbehandlung)

- Lösungsmittel:: 78,0 Gewichtsteile Aceton
1,5 Gewichtsteile Dimethylformamid
- Emulgator:: 0,5 Gewichtsteile Alkylarylpolyglykolether .

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von ≥ 80 % bei einer Aufwandmenge von 100 g/ha:
Bsp. Nr. A.4, A.5, A. 9, A.7, A.13, A.11, A.17, A.15, A. 8, A. 12

### Beispiel Nr. 3

### Nilaparvata lugens-Test (hydroponische Behandlung)

- Lösungsmittel:: 78 Gewichtsteile Aceton
1,5 Gewichtsteile Dimethylformamid
- Emulgator:: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Die Wirkstoffzubereitung wird in Wasser pipettiert. Die angegebene Konzentration bezieht sich auf die Wirkstoffmenge pro Volumeneinheit Wasser (mg/1 = ppm), anschließend wird mit der Braunrückigen Reiszikade *(Nilaparvata lugens)* infiziert.

Nach der gewünschten Zeit wird die Wirkung in % bestimmt. Dabei bedeutet 100 %, dass alle Reiszikaden abgetötet wurden; 0 % bedeutet, dass keine Reiszikaden abgetötet wurden.

Bei diesem Test zeigen z. B. die folgenden Verbindungen der Herstellungsbeispiele eine Wirkung von ≥ 80 % bei einer Aufwandmenge von 100 g/ha:
Bsp. Nr. A 6

### Beispiel Nr. 4

### 1. Herbizide Wirkung im Vorauflauf

Samen von mono- bzw. dikotylen Unkraut- bzw. Kulturpflanzen werden in Holzfasertöpfen in sandigem Lehmboden ausgelegt und mit Erde abgedeckt. Die in Form von benetzbaren Pulvern (WP) oder Emulsionskonzentraten (EC) formulierten Testverbindungen werden dann als wässrige Suspension bzw. Emulsion mit einer Wasseraufwandmenge von umgerechnet 600 1/ha unter Zusatz von 0,2 % Netzmittel in unterschiedlichen Dosierungen auf die Oberfläche der Abdeckerde appliziert. Nach Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100 % Wirkung = Pflanzen sind abgestorben, 0 % Wirkung = wie Kontrollpflanzen).

Folgende Verbindungen zweigen im Vorauflauf mit 320 g/ha a.i. gegen Alopecurus myosuroides, Avena fatua, Echinocloa crus-galli, Lolium multiflorum und Setaria viridis eine Wirkung von ≥ 90 %: Bsp. A-1, A-2, A-3, A-4, A-5, A-6, A-7, A-9, A-10, A-11, A-13, A-14, A-15, A-16, A-17, A-18, A-19, A-20, A-21

### 2. Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die als Spritzpulver (WP) oder Emulsionskonzentrat (EC) formulierten Testverbindungen werden in verschiedenen Dosierungen mit einer Wasseraufwandmenge von umgerechnet 600 1/ha unter Zusatz von 0,2 % Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100 % Wirkung = Pflanzen sind abgestorben, 0 % Wirkung = wie Kontrollpflanzen).

Folgende Verbindungen zeigen im Nachlauf mit 80 g/ha a.i. gegen Alopecurus myosuroides, Avena fatu, Echinochloa crus-galli, Lolium multiflorum und Setaria viridis eine Wirkung von ≥ 90 %: Bsp. A-1, A-2, A-3, A-4, A-5, A-6, A-7, A-8, A-9, A-10, A-11, A-12, A-13, A-14, A-15, A-16, A-17, A-18, A-19, A-20, A-21

## Patentansprüche

1. Verbindungen der Formel (I) 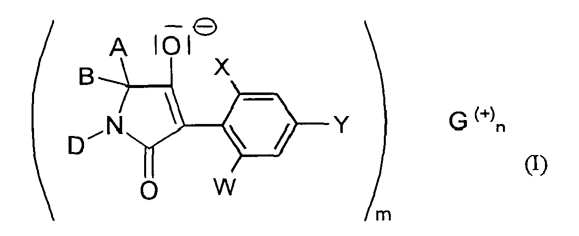 in welcher
X für Alkoxy, Halogenalkoxy, Alkoxyalkyloxy, Alkoxy-bis-alkyloxy oder gegebenenfalls substituiertes Cycloalkyl-alkandyl-oxy, welches gegebenenfalls durch Heteroatome unterbrochen sein kann, steht,
W für Alkyl steht,
Y für Chlor, Brom oder Iod steht,
G für ein Metallionenäquivalent steht,
m für die Zahl 1 oder 2 steht,
n für die Zahl 1 oder 2 steht,
A für Wasserstoff, jeweils gegebenenfalls durch Halogen substituiertes Alkyl, Alkenyl, Alkoxyalkyl, Alkylthioalkyl oder Cycloalkyl steht,
B für Wasserstoff, Alkyl oder Alkoxyalkyl steht, oder
A und B gemeinsam mit dem Kohlenstoffatom an das sie gebunden sind, für einen gesättigten oder ungesättigten, gegebenenfalls mindestens ein Heteroatom enthaltenden unsubstituierten oder substituierten Cyclus stehen,
D für Wasserstoff oder einen gegebenenfalls substituierten Rest aus der Reihe Alkyl, Alkenyl, Alkinyl, Alkoxyalkyl, gesättigtes oder ungesättigtes Cycloalkyl, in welchem gegebenenfalls eines oder mehrere Ringglieder durch Heteroatome ersetzt sind, Arylalkyl, Aryl, Hetarylalkyl oder Hetaryl steht oder
A und D gemeinsam mit den Atomen an die sie gebunden sind für einen gesättigten oder ungesättigten und gegebenenfalls mindestens ein Heteroatom enthaltenden, im A,D-Teil unsubstituierten oder substituierten Cyclus stehen.

2. Verfahren zur Herstellung von Verbindungen der Formel (I) gemäß Anspruch 1 **dadurch gekennzeichnet, dass** man zum Erhalt von:
(A) Verbindungen der Formel (I) 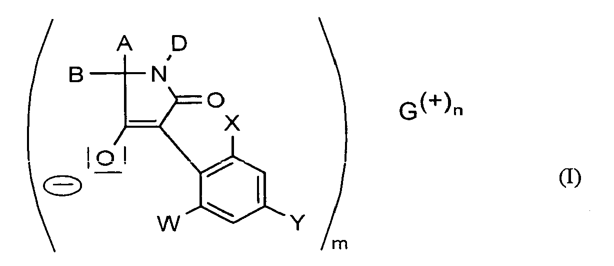 in welcher
A, B, D, m, n, W, X und Y die oben angegebenen Bedeutungen haben,
Verbindungen der Formel (II) 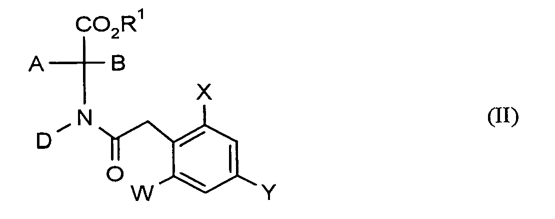 in welcher
A, B, D, W, X und Y die oben angegebenen Bedeutungen haben,
und
R¹ für Alkyl steht,
in Gegenwart eines Verdünnungsmittels und in Gegenwart einer Metallbase intra-molekular kondensiert,
(B) Verbindungen der oben gezeigten Formel (I), in welcher A, B, D, m, n, W, X und Y die oben angegebenen Bedeutungen haben, Verbindungen der Formel (I'), 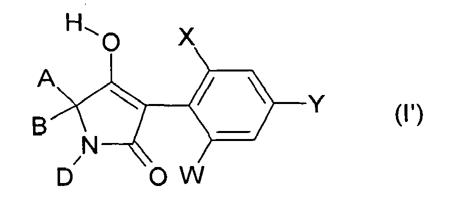 in welchen A, B, D, W, X und Y die oben angegebenen Bedeutungen haben, jeweils
mit Metallverbindungen der Formeln (III) oder (IV)
G(OR²)ₙ (III) ,
G(H)ₙ (IV)
in welchen
G für ein ein- oder zweiwertiges Metall,
n für die Zahl 1 oder 2 und
R² für Wasserstoff oder Alkyl steht,
gegebenenfalls in Gegenwart eines Verdünnungsmittels umsetzt.

3. Mittel zur Bekämpfung von Schädlingen und/oder unerwünschten Pflanzenwuchs, **gekennzeichnet durch** einen Gehalt an mindestens einer Verbindung der Formel (I) gemäß Anspruch 1.

4. Verfahren zur Bekämpfung von tierischen Schädlingen und/oder unerwünschtem Pflanzenbewuchs, **dadurch gekennzeichnet, dass** man Verbindungen der Formel (I) gemäß Anspruch 1 auf Schädlinge, unerwünschtem Pflanzenwuchs, und/oder ihren Lebensraum einwirken lässt.

5. Verwendung von Verbindungen der Formel (I) gemäß Anspruch 1 zur Bekämpfung von tierischen Schädlingen und/oder unerwünschtem Pflanzenbewuchs.

6. Verfahren zur Herstellung von Mitteln zur Bekämpfung von Schädlingen und/oder unerwünschtem Pflanzenbewuchs, **dadurch gekennzeichnet, dass** man Verbindungen der Formel (I) gemäß Anspruch 1 mit Streckmitteln und/oder oberflächenaktiven Stoffen vermischt.

7. Verwendung von Verbindungen der Formel (I) gemäß Anspruch 1 zur Herstellung von Mitteln zur Bekämpfung von Schädlingen und/oder unerwünschtem Pflanzenwuchs.
